# FASCICULE DE BREVET EUROPEEN

(11) **EP 2 138 490 B1**
(45) Date de publication et mention de la délivrance du brevet: **31.08.2011**
(21) Numéro de dépôt: 08104076.8
(22) Date de dépôt: 23.05.2008
(51) Int. Cl.: C07D 403/04

(54) **Nouveau procédé de synthèse de fluoroquinolones**
Neues Syntheseverfahren für Fluorquinolone
New method for synthesis of fluoroquinolones

(30) Priorité: 24.05.2007 FR 0755240
(43) Date de publication de la demande: 30.12.2009
(73) Titulaire: Biocodex, 94250 Gentilly (FR)
(72) Inventeur: Berthon-Cédille, Laurence, 60490 Ricquebourg (FR); Leguern, Marie-Emmanuelle, 60200 Compiegne (FR); Renaud, Gilles, 92400 Courbevoie (FR); Tombret, Francis, 60350 Trosly-Breuil (FR)
(74) Mandataire: Vial, Lionel

(56) Documents cités:
- EP-A- 0 657 448
- WO-A-03/010144
- BOUZARD D ET AL: "FLUORONAPHTHYRIDINES AND QUINOLONES AS ANTIBACTERIAL AGENTS. 1. SYNTHESIS AND STRUCTURE-ACTIVITY RELATIONSHIPS OF NEW 1-SUBSTITUTED DERIVATIVES" JOURNAL OF MEDICINAL CHEMISTRY, AMERICAN CHEMICAL SOCIETY. WASHINGTON, US, vol. 32, no. 3, 1989, pages 537-542, XP001106566 ISSN: 0022-2623

## Description

La présente invention concerne un procédé de préparation de fluoroquinolones.

Les fluoroquinolones sont des antibiotiques obtenus par synthèse chimique présentant un large spectre antibactérien. Ces composés inhibent la topo-isomérase Il ou DNA-gyrase et ainsi la réplication et la transcription de l'ADN des bactéries.

Comme exemple de fluoroquinolones, on peut citer notamment la loméfloxacine, la ciprofloxacine, la norfloxacine et la péfloxacine, dont les structures sont représentées ci-dessous. 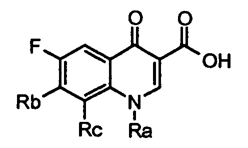 Loméfloxacine : Rₐ = éthyle, R_{b} = 3-méthyl-1-pipérazinyle, R_{c}=F; Ciprofloxacine :
Rₐ = cyclopropyle, R_{b} = 1-pipérazinyle, R_{c}= H ; Norfloxacine : Rₐ = éthyle,
R_{b} = 1-pipérazinyle, R_{c}= H ; Péfloxacine : Rₐ = éthyle, R_{b} = 4-méthyl-1-pipérazinyle, R_{c}= H.

Plusieurs procédés de synthèse de ces composés sont rapportés dans la littérature (US 4,146,719 ; FR 2 555 584 ; Hiroshi Koga et al., J. Med. Chem., 1980, 23, 1358-1363 ; Domagala et al., J. Med. Chem, 1991, 34, 1142-1154). Ces procédés impliquent généralement la mise en oeuvre de deux étapes, dont notamment une étape a) d'hydrolyse d'ester carboxylique (3) en l'acide correspondant (2) suivie d'une étape b) de substitution nucléophile d'un atome d'halogène, notamment Cl ou F, par un agent précurseur du groupe R_{b}, notamment un dérivé pipérazinyle, ce par quoi on obtient la fluoroquinolone souhaitée (1) (Schéma I). Les deux réactions chimiques, hydrolyse et substitution peuvent être également mises en oeuvre dans un ordre inverse, substitution nucléophile du noyau halogène puis hydrolyse de l'ester.

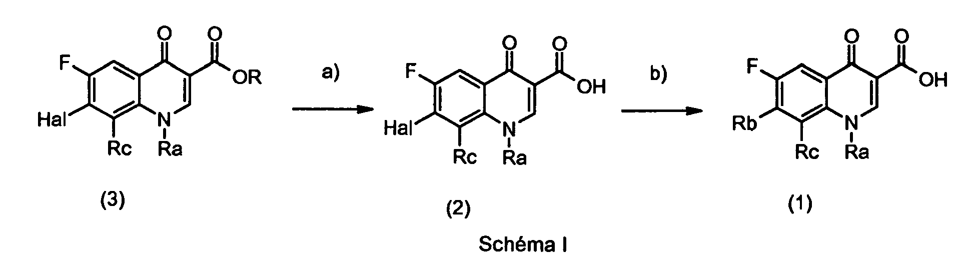

L'étape b) est généralement réalisée en présence d'un solvant organique tel que la pyridine ou l'acétonitrile au reflux, ou encore le DMF, éventuellement en présence d'une base organique telle que la triéthylamine (Domagala et al., J. Med. Chem, 1991, 34, 1142-1154).

Un procédé de préparation de fluoroquinolones impliquant une étape b) dans l'eau a récemment été rapporté dans la littérature (M. Saeed Abaee et al., Heterocyclic Communications, Vol. 11, No. 5, 2005). Cependant, cette réaction s'accompagne de la formation d'une quantité importante d'isomère de position. De plus, il est nécessaire de chauffer le milieu réactionnel pendant plusieurs heures à une température élevée.

Il a maintenant été mis au point un nouveau procédé de préparation de fluoroquinolones en un nombre limité d'étapes. Plus spécifiquement, selon ce procédé, les étapes a) et b) ci-dessus sont avantageusement réalisées en une seule étape.

En outre, ce procédé est réalisé dans l'eau ce qui constitue un avantage économique et écologique important.

Enfin, ce procédé permet d'accéder à des fluoroquinolones diversement substituées avec des rendements et une pureté élevés. Avantageusement, le procédé selon l'invention permet d'obtenir des rendements supérieurs à 90 % voire à 96 %.

La présente invention concerne ainsi un procédé de préparation d'un composé de formule (I) : 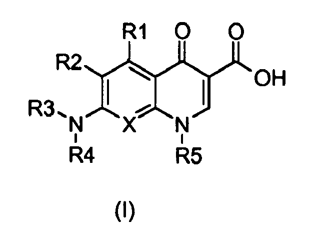 ou d'un hydrate ou d'un sel d'addition acide ou basique de celui-ci,
dans laquelle :
R₁ représente un groupement choisi parmi Hydrogène, Alkyle, ou NRR' ;
R₂ représente un atome de fluor ;
R₃, R₄ identiques ou différents
   - représentent indépendamment un groupement Hydrogène, Alkyle, Cycloalkyle, Hydroxyle, Aralkyle, lesdits groupes Alkyle, Cycloalkyle, Aralkyle, pouvant être éventuellement substitués par un ou plusieurs groupes Hydroxyles, ou NRR' ;
      ou
   - forment ensemble avec l'atome d'azote auquel ils sont attachés un groupement hétérocyclyle éventuellement substitué par un ou plusieurs groupes choisis parmi Alkyle, Hydroxyle, Alkoxy, -C(=O)Alkyle, NRR', =NOR, Aralkyle, Aryle, Hétéroaryle,
      lesdits groupes Alkyle, Aryle et Hétéroaryles pouvant être éventuellement substitués par un ou plusieurs groupes choisis parmi Alkyle, Halogène, Perfluoroalkyle, Alkoxy, NRR';
R₅ représente un groupement choisi parmi Hydrogène, Alkyle, Cycloalkyle, Aryle, NR(CHO) ou NRR', lesdits groupes Alkyles et Aryles pouvant être éventuellement substitués par un ou plusieurs groupes choisis parmi Halogène ou Hydroxyle ;
X représente un groupement CR₈ ou un atome d'azote ;
R₈ représente un groupement choisi parmi Hydrogène, Halogène, Alkyle, ou Alkoxy, ou forme avec R₅ un groupement Hétérocyclyle, éventuellement substitué par un ou plusieurs groupes Alkyle ;
R, R' identiques ou différents représentent indépendamment un groupe Hydrogène ou Alkyle ;
ledit procédé comprenant :
i) la réaction d'un composé de formule (II) 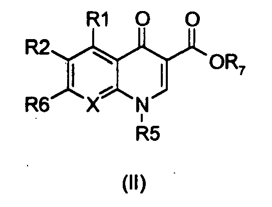 dans laquelle
   R₁, R₂, R₅ et X sont tels que définis ci-dessus,
   R₆ représente un atome d'Halogène ;
   R₇ représente un groupement Alkyle ;
   avec une amine de formule R₃R₄NH dans l'eau en présence d'une base minérale; et éventuellement
ii) la récupération du composé de formule (I) obtenu.

Selon la présente invention, les radicaux Alkyle représentent des radicaux hydrocarbonés saturés, en chaîne droite ou ramifiée, de 1 à 20 atomes de carbone, de préférence de 1 à 5 atomes de carbone. On peut notamment citer, lorsqu'ils sont linéaires, les radicaux méthyle, éthyle, propyle, butyle, pentyle, hexyle, octyle, nonyle, décyle, dodécyle, hexadécyle, et octadécyle. On peut notamment citer, lorsqu'ils sont ramifiés ou substitués par un ou plusieurs radicaux alkyle, les radicaux isopropyle, tert-butyle, 2-éthylhexyle, 2-méthylbutyle, 2-méthylpentyle, 1-méthylpentyle et 3-méthylheptyle.

Les radicaux Alkoxy selon la présente invention sont des radicaux de formule -O-Alkyle, l'alkyle étant tel que défini précédemment.

Parmi les atomes d'Halogène (Hal), on cite plus particulièrement les atomes de fluor, de chlore, de brome et d'iode, de préférence le chlore ou le fluor.

Le radical Cycloalkyle est un radical hydrocarboné mono-, bi- ou tri- cyclique saturé ou partiellement insaturé, non aromatique, de 3 à 10 atomes de carbone, tel que notamment le cyclopropyle, cyclopentyle, cyclohexyle ou adamantyle, ainsi que les cycles correspondants contenant une ou plusieurs insaturations.

Aryle désigne un système aromatique hydrocarboné, mono- ou bicyclique de 6 à 10 atomes de carbone. Parmi les radicaux Aryle, on peut notamment citer le radical phényle ou naphtyle, plus particulièrement substitué par au moins un atome d'halogène.

Aralkyle désigne un groupe aryl-alkyl- où aryl et alkyl sont tels que définis ci-dessus. Parmi les radicaux Aralkyle, on peut notamment citer le radical benzyle ou phénéthyle.

Les radicaux Hétéroaryles désignent les systèmes aromatiques comprenant un ou plusieurs hétéroatomes choisis parmi l'azote, l'oxygène ou le soufre, mono-ou bicyclique, de 5 à 10 atomes de carbone. Parmi les radicaux Hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes condensés à un noyau phényle. Les groupes Hétéroaryle préférés comprennent le thiényle, le pyrrolyle, le quinoxalinyle, le furanyle, l'imidazolyle, l'indolyle, l'isoxazolyle, l'isothiazolyle, le pyrazinyle, le pyridazinyle, le pyrazolyle, le pyridyle, le pyrimidinyle, le quinazolinyle, le quinolinyle, le thiazolyle, le carbazolyle, le thiadiazolyle, et les groupes condensés à un noyau phényle, et plus particulièrement le quinolynyle, le carbazolyle, le thiadiazolyle.

Les radicaux Hétérocyclyles désignent les systèmes mono- ou bicycliques, saturés ou partiellement insaturés, non aromatiques, de 5 à 10 atomes de carbone, comprenant un ou plusieurs hétéroatomes choisis parmi N, O ou S. Parmi les Hétérocyclyles, on peut notamment citer l'oxiranyle, l'aziridinyle, le tétrahydrofuranyle, le dioxolanyle, le pyrrolidinyle, le pyrazolidinyle, l'imidazolidinyle, le tétrahydrothiophényle, le dithiolanyle, le thiazolidinyle, le tétrahydropyranyle, le dioxanyle, le morpholinyle, le pipéridyle, le pipérazinyle, le tétrahydrothiopyranyle, le dithianyle, le thiomorpholinyle, le dihydrofuranyle, le 2-imidazolinyle, le 2,-3-pyrrolinyle, le pyrazolinyle, le dihydrothiophényle, le dihydropyranyle, le pyranyle, le tétrahydropyridyle, le dihydropyridyle, le tétrahydropyrinidinyle, le dihydrothiopyranyle, et les groupes correspondants issus de la fusion avec un noyau phényle, et plus particulièrement les cycles pipérazinyle, pyrrolidinyle, pipéridinyle et morpholinyle.

Les composés de l'invention de formule (I) définis tels que précédemment possédant une fonction suffisamment acide ou une fonction suffisamment basique ou les deux, peuvent inclure les sels correspondants d'acide organique ou minéral ou de base organique ou minérale, de préférence pharmaceutiquement acceptables. Ils peuvent se présenter également sous forme zwittérionique.

Les sels d'additions acides sont formés avec les composés utiles selon l'invention dans lesquels une fonction de base tels qu'un groupe amino, alkylamino ou dialkylamino est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Parmi les acides adaptés pour l'usage dans la préparation de ces sels on trouve acide chlorhydrique, acide bromhydrique, acide phosphorique, acide sulfurique, divers acides carboxyliques et sulfoniques organiques, tels que acide acétique, acide citrique, acide propionique, acide succinique, acide benzoïque, acide tartrique, acide fumarique, acide mandélique, acide ascorbique, acide malique, acide méthanesulfonique, acide toluène-sulfonique, acides gras.

Les sels d'addition de base peuvent être formés lorsque le composé utile selon l'invention contient un groupe carboxyle, ou un bioisostère suffisamment acide. Les bases qui peuvent être utilisées pour préparer les sels d'addition de base comprennent de préférence celles qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine et autres.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvants aqueux/organiques, en utilisant des solvants organiques tels que dioxane, tétrahydrofuranne ou méthanol.

Les composés de formule (II) utiles selon l'invention sont ceux pour lesquels R₁ représente de préférence un atome d'hydrogène.

De préférence, R₃, R₄ forment ensemble avec l'atome d'azote auquel ils sont attachés un groupement hétérocyclyle, en particulier un groupe pipérazinyle, pipéridinyle, pyrrolidinyle, ou morpholinyle.

De préférence, R₃, R₄ forment ensemble avec l'atome d'azote auquel ils sont attachés un groupe pipérazinyle.

De préférence, R₅ représente un groupe Alkyle, Cycloalkyle ou Aryle, notamment un groupe éthyle, cyclopropyle ou un groupe phényle substitué par un ou plusieurs atomes de fluor.

De préférence, R₈ représente H, F, Cl ou OCH₃.

Selon une variante, R₈ forme avec R₅ un groupe hétérocyclyle Het1: 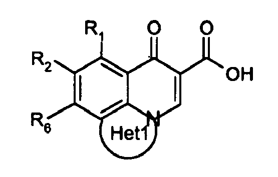 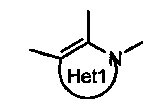 représente de préférence un groupement hétérocyclyle comprenant préférentiellement de 5 à 7 chaînons, notamment 6 chaînons, plus préférentiellement choisis parmi : 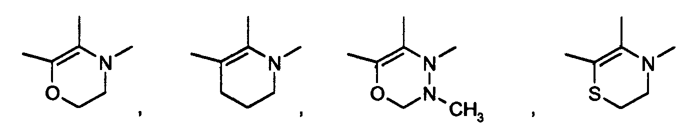 éventuellement substitué par un ou plusieurs groupes alkyles.

De préférence, R₆ représente un atome de fluor, de chlore ou de brome, plus préférentiellement un atome de fluor.

Plus préférentiellement, les composés de formule (I) peuvent être choisis parmi:
- Loméfloxacine
- Norfloxacine
- Ciprofloxacine
- Péfloxacine
- Grépafloxacine ou toméfloxacine
- Ofloxacine ou Lévofloxacine
- Flérofloxacine
- Sarafloxacine
- Gatifloxacine
- Enrofloxacine
- Difloxacine
- Témafloxacine
- Amifloxacine
- Sitafloxacine
- Danofloxacine
- Clinafloxacine
- Balofloxacine
- Moxifloxacine
- Sparfloxacine
- Nadifloxacine
- Marbofloxacine
- Lofloxacine
- Rufloxacine
- Enofloxacine
- Gémifloxacine
- Trovafloxacine
- Tosufloxacine.

De préférence, l'amine de formule R₃R₄NH mise en oeuvre à l'étape i) représente 1 à 5 équivalents, plus préférentiellement 2 à 4 équivalents, notamment 3 équivalents par rapport au nombre de moles du composé de formule (II). De façon inattendue, il a en effet été démontré que l'utilisation d'un nombre croissant d'équivalents d'amine permet d'accélérer la réaction pour atteindre, à partir de 3 équivalents, un taux de conversion de plus de 90% après seulement quelques heures de réaction à température.

l'étape i) est réalisée en présence d'une base minérale, plus préférentiellement en présence d'un carbonate ou d'un hydrogénocarbonate de métal alcalin, notamment de K₂CO₃, KHCO₃, Na₂CO₃, ou NaHCO₃, K₂CO₃ étant particulièrement préféré. Avantageusement, K₂CO₃ permet d'accélérer la cinétique de la réaction et conduit à un taux d'impureté très faible.

La quantité de base minérale présente dans le milieu n'est pas critique. Elle peut varier entre 1,2 et 5 équivalents en moles par rapport au composé de formule (II), et est de préférence comprise entre 3 et 5.

La réaction peut avoir lieu dans une large gamme de températures, notamment entre 20°C et 200°C. De préférence, la ré action est réalisée à une température comprise entre 60°C et 120°C, plus préférentiellement entre 90°C et 100°C.

Le temps requis pour la réaction peut varier considérablement, selon de nombreux facteurs, notamment la température de réaction et la nature des réactifs. Généralement une durée d'environ 3 heures à environ 12 heures est suffisante.

Le composé ainsi préparé peut être récupéré à partir du mélange de la réaction par les moyens traditionnels. Par exemple, les composés peuvent être récupérés soit par cristallisation à pH contrôlé ou bien par addition d'un solvant dans lequel les produits ne sont pas solubles, soit par extraction du milieu réactionnel avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention puissent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention englobe des isomères géométriques individuels, des stéréoisomères et des mélanges de ceux-ci, incluant des mélanges racémiques, de composés de formule (I) ci-dessus. Ces types d'isomères peuvent être séparés de leurs mélanges, par l'application ou l'adaptation de procédés connus, par exemple des techniques de chromatographie ou des techniques de recristallisation préférentielle, ou ils sont préparés séparément à partir des isomères appropriés de leurs intermédiaires.

Le procédé selon l'invention peut comprendre en outre une étape subséquente à l'étape i) ou ii), dans laquelle le composé de formule (I) obtenu est mis à réagir avec une solution d'acide chlorhydrique, de manière à obtenir le chlorhydrate correspondant.

Les composés de formule générale (II) sont disponibles commercialement et/ou peuvent être préparés par application ou adaptation de toute méthode connue en soi et/ou à la portée de l'homme du métier, notamment celles décrites par R.C. Larock dans Comprehensive Organic Transformations, VCH Pub., 1989, ou par application ou adaptation des procédés décrits dans les exemples qui suivent.

Les composés de formule (II) peuvent être notamment préparés à partir de composés de formule (III) : 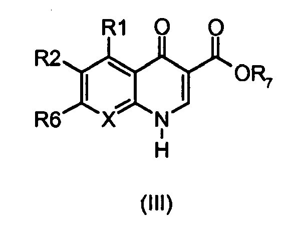 dans laquelle R₁, R₂, R₆, X et R₇ sont tels que définis ci-dessus dans la formule générale (II).

A titre d'exemple, on fait réagir un composé de formule (III) avec un composé R₅-Hal, en présence d'une base organique ou minérale telle que K₂CO₃ ou NaH, dans un solvant aprotique polaire tel que le DMF, à une température d'environ 100°C.

Les composés de formule (III) pour lesquels X=CR₈ peuvent être préparés à partir de composés de formule (IV) : 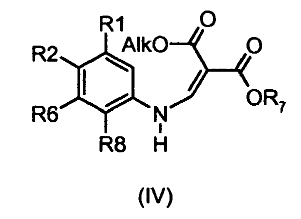 dans laquelle R₁, R₂, R₆, R₈ et R₇ sont tels que définis ci-dessus dans la formule générale (III).

A titre d'exemple, la cyclisation intramoléculaire est réalisée dans Ph₂O en chauffant à une température d'environ 230°C.

Les composés de formule (IV) peuvent être préparés à partir de composés de formule (V) : 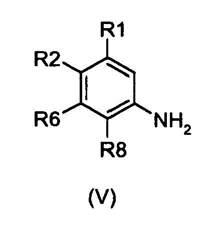 dans laquelle R₁, R₂, R₆ et R₈ sont tels que définis dans la formule générale (IV).

Les composés de formule (IV) peuvent être obtenus notamment en couplant les composés de formule (V) avec des composés de formule (VI) : 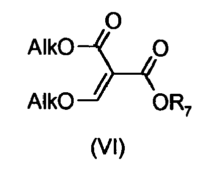 dans laquelle les groupes Alk désignent indépendamment un groupe alkyle et R₇ est tel que défini dans la formule (IV). Ce couplage peut être réalisé par exemple dans le toluène, à 105°C.

Selon un autre aspect, les composés de formule (II) peuvent être préparés à partir des composés de formule (VII) : 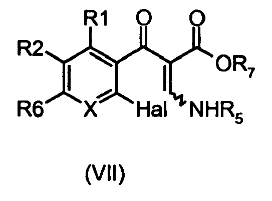 dans laquelle R₁, R₂, R₅, R₆, R₇ et X sont tels que définis dans la formule générale (II), et Hal représente un atome d'halogène.

Les composés de formule (VII) peuvent être préparés à partir des composés de formule (VIII) : 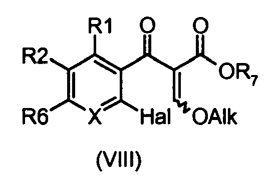 dans laquelle R₁, R₂, R₆, R₇, X et Hal sont tels que définis dans la formule générale (VII) et Alk représente un groupe alkyle.

Cette étape peut être réalisée en faisant réagir une amine R₅NH₂ sur un composé de formule (VIII) dans un solvant tel que CH₂Cl₂ ou l'EtOH.

Les composés de formule (VIII) peuvent être préparés à partir des composés de formule (IX) : 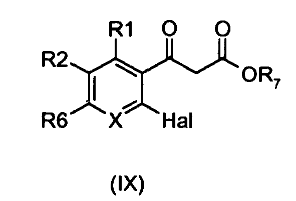 dans laquelle R₁, R₂, R₆, R₇, X et Hal sont tels que définis dans la formule générale (VIII).

A titre d'exemple, cette réaction peut être réalisée en faisant réagir un composé HC(OAlk)₃ sur un composé de formule (IX) dans l'anhydride acétique.

Les composés de formule (IX) peuvent être préparés à partir des composés de formule (X) : 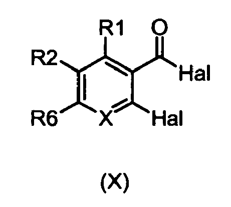 dans laquelle R₁, R₂, R₆ et X sont tels que définis dans la formule générale (VII) et les groupes Hal représentent indépendamment un atome d'halogène.

A titre d'exemple, cette réaction peut être réalisée en faisant réagir un composé de formule R₇O(CO)CH₂COO en présence de MgCl₂ et de triéthylamine (TEA) dans l'acétonitrile.

Des schémas illustratifs des procédés de préparation des composés de formule (II) utiles selon l'invention sont représentés ci-après.

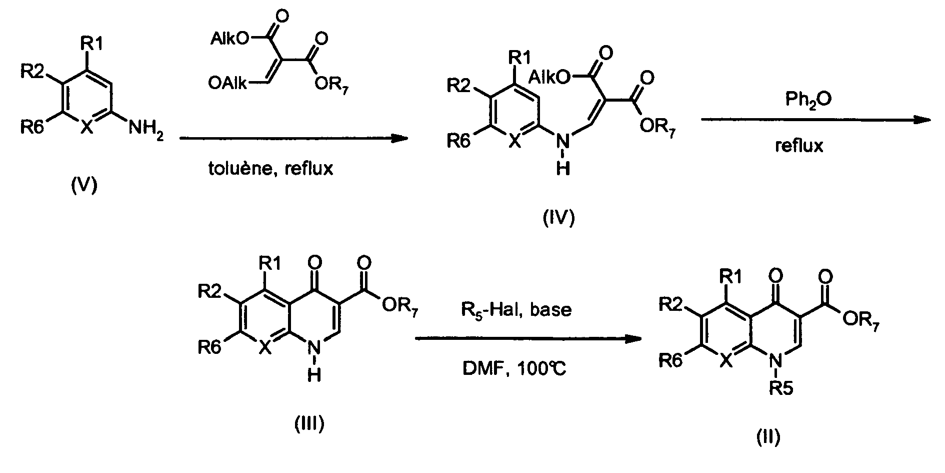

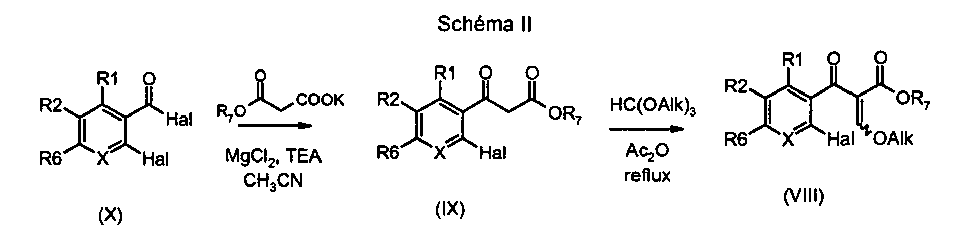

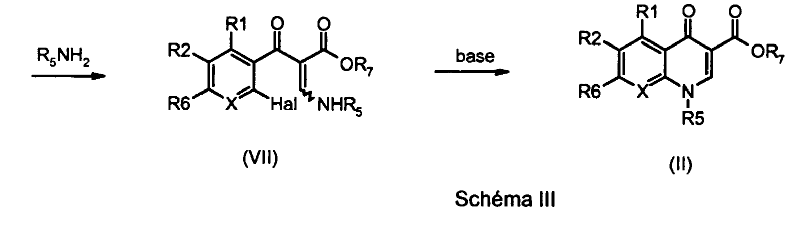

Dans les réactions décrites ci-dessus, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Green et P.G.M. Wuts dans Protective Groups dans Organic Chemistry , John Wiley and Sons, 1991 ; J.F.W. McOmie in Protective Groups dans Organic Chemistry, Plenum Press, 1973.

Les produits de bases ou les réactifs utilisés sont disponibles commercialement et/ou peuvent être préparés par l'application ou l'adaptation de procédés connus, par exemple des procédés tels que décrits dans les exemples ou leurs équivalents chimiques évidents.

Les exemples suivants illustrent l'invention, sans toutefois la limiter. Les produits de départs utilisés sont des produits connus ou préparés selon des modes opératoires connus.

Les pourcentages sont exprimés en poids, sauf mention contraire.

### EXEMPLES

### Exemple 1 : Procédé de préparation de l'acide 1-éthyl-6,8-difluoro-1,4-dihydro-7-(3-méthyl-1-pipérazinyl)-4-oxoquinoline-3-carboxylique (loméfloxacine).

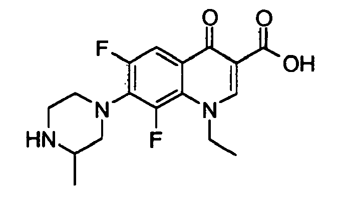

Dans un tricol de 1l, ajouter 300 ml d'eau, agiter puis additionner 50 g de 1-éthyl-6,7,8-trifluoro-1,4-dihydro-4-oxoquinoline-3-carboxylate d'éthyle, 50 g de carbonate de potassium et 50 g de 2-méthylpipérazine. Chauffer ensuite le milieu réactionnel à 90-95°C en masse pendant minimum 10 h en contrôlant l'évolution de la réaction par CCM. Lorsque la réaction est terminée, refroidir le milieu réactionnel à température ambiante et ajuster le pH à environ 7,5 par addition d'acide chlorhydrique à 37% (le produit précipite au cours de la coulée). Agiter cette suspension 3 h, réajuster le pH si nécessaire, puis essorer. Réempater successivement avec 2 x 50 ml d'eau puis 2 x 50 ml d'acétone et clarcer avec 50 ml d'acétone. Après séchage en étuve à 50°C, on obtient 56,5 g de cristaux blancs.( Rdt ~ 96 %).

### Loméfloxacine : Acide 1-éthyl-6,8-difluoro-1,4-dihydro-7-(3-méthyl-1-pipéra-zinyl) -4-oxoquinoline-3-carboxylique; RN [98079-51-7].

**pF** : 238°C [litt. : 239-240,5°C; Merck Index 14th, 5562].
**RMN ¹H** (5 % NaOD /D₂O) en ppm : 1,07 (d; ³J = 6,0 Hz; 3H; CH₃ pipérazino); 1,42 (t; ³J = 6,6 Hz; 3H; CH₃ éthyle); 2,8 à 3,1 (m; 4H; 2 CH₂ pipérazino); 3,1 à 3,4 (m; 3H; CH pipérazino + CH₂ pipérazino); 4,36 (m; 2H; CH₂ éthyle); 7,66 (dd; ³J_{H-F6} = 12,6 Hz et ⁵J_{H-F8}= 1,8 Hz; 1 H; H-5); 8,32 (s; 1 H; H-2).
**RMN ¹³C** (5 % NaOD /D₂O) en ppm : 17,93 (d; ⁵J_{C-F8} = 4,6 Hz; CH₃ éthyle); 20,57 (s; CH₃ pipérazino); 47,51 - 52,89 - 60,00 (3 s; 3 CH₂ pipérazino); 52,70 (s; CH pipérazino); 56,02 (d; ⁴J_{C-F8} = 16,8 Hz; CH₂ éthyle); 109,59 (d; ²J_{C-F6} = 23,7 Hz; CH-5); 118,56 (s; C-3); 125,48 (d; ²J_{C-F8} = 7,7 Hz; C-8a); 128,92 (d; 3J_{C-F6} = 6,1 Hz; C-4a); 134,77 (t; ²J_{C-F6} ~ ²J_{C-F8} = 14,5 Hz; C-7); 147,9 (dd; ¹J_{C-F8} = 248,2 Hz et ³J_{C-F6} = 6,5 Hz; C-8); 152,42 (s; CH-2); 156,30 (dd; ¹J_{C-F6} = 245,9 Hz et ³J_{C-F8} = 6,1 Hz; C-6); 174,19 (s; CO₂H); 176,45 (s; CO).
**IR** (ATR) en cm⁻¹: 3600 à 3200 (valence O-H lié type dimère et valence N-H amine secondaire), 3100 à 2800 et 2800 à 2200 (valence O-H acide type dimère et valence C-H aromatiques ou aliphatiques), 3053 (valence C-H aromatiques), 1723 (valence C=O acide type dimère), 1614 (valence C=O cétone conjuguée et valence asymétrique ion carboxylate O-C-O), 1475 (valence C=C aromatiques), 1338 (valence symétrique ion carboxylate OC-O), 1280 (valence C-F aromatique), 1250 (valence C-O acide type dimère), 930 (H-O déformation hors du plan)

A titre d'exemples, les composés suivants sont préparés selon le mode opératoire de l'exemple 1 :

### Acide 1-éthyl-6,8-difluoro-1,4-dihydro-7-[(2-hydroxypropyl)amino]-4-oxoquinoline-carboxylique; RN = [724737-42-2]

**pF** : 195,5°C.
**RMN ¹H** (5 % NaOD /D₂O) en ppm : 1,21 (d; ³J = 6,2 Hz; 3H; CH₃ propyle); 1,37 (t; ³J = 6,6 Hz; 3H; CH₃ éthyle); 3,2 à 3,45 (m; 2H; CH₂ propyle); 3,85 à 4,05 (m; 1H; CH propyle); 4,15 à 4,4 (m; 2H; CH₂ éthyle); 7,51 (dd; ³J_{H-F6} = 12,8 Hz et ⁵J_{C-F8} = 1,6 Hz; 1 H; H-5); 8,26 (s; 1 H; H-2).
**RMN ¹³C** (5 % NaOD /D₂O) en ppm : 13,27 (d; ⁵J_{C-F8} = 4,6 Hz; CH₃ éthyle); 17,45 (s; CH₃ propyle); 49,52 (s; CH₂ propyle); 51,12 (d; ⁴J_{C-F8} 16,8 Hz; CH₂ éthyle); 65,16 (s; CH propyle); 104,42 (d; ²J_{C-F6} = 21,4 Hz; CH-5); 113,53 (s; C-3 ); 115,71 (d; ²J_{C-F8} = 6,9 Hz; C-8a); 124,12 (d; ³J_{C-F6} = 5,4 Hz; C-4a); 128,53 (pseudo t; ²J_{C-F} = 13,8 et 14,5 Hz; C-7); 136,95 (dd; ¹J_{C-F8} = 239,8 Hz et ³J_{C-F6} = 6,9 Hz; C-8); 147,14 (s; CH-2); 147,32 (dd; ¹J_{C-F6} = 242,1 Hz et ³J_{C-F8} = 7,7 Hz; C-6); 169,97 (s; CO₂H); 172,08 (s; CO).
**IR** (ATR) en cm⁻¹ : 3360 (valence O-H lié type dimère), 3288 (valence N-H amine secondaire libre), 3150 à 2800 et 2800 à 2200 (valence O-H acide type dimère et valence C-H aromatiques et aliphatiques), 1695 (valence C=O acide type dimère), 1625 (valence C=O cétone conjuguée et valence asymétrique ion carboxylate O-C-O), 1488 (valence C=C aromatiques).
Acide 8-chloro-1-éthyl-6-fluoro-1,4-dihydro-7-(3-méthyl-1-pipérazinyl)-4-oxoquinoline-3-carboxylique; RN = [111234-01-6].

**pF :** 208-210°C.
**RMN ¹H** (5 % NaOD /D₂O) en ppm : 1,03 (d; ³J = 6,0 Hz; 3H; CH₃ pipérazino); 1,33 (t; ³J = 7,2 Hz; 3H; CH₃ éthyle); 2,60 à 3,25 (m; 7H; CH + 3 CH₂ pipérazino); 4,48 (q; ³J = 7,0 Hz; 2H; CH₂ éthyle); 7,68 (d; ³J _{H-F} = 12,6 Hz; 1H; H-5); 8,38 (s; 1H; H-2).
**RMN ¹³C** (5 % NaOD /D₂O) en ppm : 13,16 (s; CH₃ éthyle); 15,93 (s; CH₃ pipérazino); 42,95 - 48,38 - 50,53 - 55,55 (4 s; 3 CH₂ pipérazino + CH₂ éthyle); 48,07 (s; CH pipérazino); 109,2 (d; ²J_{C-F6} = 23,0 Hz; CH-5); 114,63 (s; C-3); 116,28 (s; C-8a); 123,47 (d; ³J_{C-F6} = 6,8 Hz; C-4a); 133,78 (s; C-8); 140,36 (d; ²J_{C-F6} = 14,5 Hz; C-7); 149,19 (s; CH-2); 153,18 (d; ¹J_{C-F6} = 248,0 Hz; C-6); 169,54 (s; CO₂H); 172,67 (s; CO).
**IR** (ATR) en cm⁻¹ : 3600 à 3200 (valence O-H lié type dimère et valence N-H amine secondaire), 3100 à 2800 et 2800 à 2200 (valence O-H acide type dimère et valence C-H aromatiques et aliphatiques), 1725 (valence C=O acide type dimère), 1630 - 1610 (valence C=O cétone conjuguée et valence asymétrique ion carboxylate O-C-O).

### Norfloxacine : Acide 1-éthyl-6-fluoro-1,4-dihydro-7-(1-pipérazinyl)-4-oxoquinoline-3-carboxylique; RN = [70458-96-7].

**pF :** 219-220°C [litt : 220-1°C, Merck Index 14th, 6700]
**RMN ¹H** (5 % NaOD /D₂O) en ppm : 1,39 (t; ³J = 7,0 Hz; 3H; CH₃ éthyle); 2 à 3,25 (m; 8H; 4 CH₂ pipérazino); 4,22 (pseudo q; ³J = 7,0 Hz; 2H; CH₂ éthyle); 6,88 (d; ⁴J_{H-F} = 6,9 Hz; 1 H; H-8); 7,79 (d; ³J_{H-F} = 13,6 Hz; 1 H; H-5); 8,37 (s; 1H; H-2).
**RMN ¹³C** (5 % NaOD /D₂O) en ppm : 16,23 (s; CH₃ éthyle); 46,89 - 53,17 (2 s; 4 CH₂ piperazino); 51,58 (s; CH₂ éthyle); 107,79 (s; CH-8); 114,22 (d; ²J_{C-F6} = 22,9 Hz; CH-5); 119,32 (s; C-3); 125,07 (d; ³J_{C-F6} = 6,9 Hz; C-4a); 138,96 (s; C-8a); 147,24 (d; ²J_{C-F6} = 11,5 Hz; C-7); 149,47 (s; CH-2). 155,46 (d; ¹J_{C-F6} = 245,9 Hz; C-6); 175,02 (s; COOH); 177,71 (s; CO).
**IR** (ATR) en cm⁻¹ : 3600 à 3200 (valence O-H lié type dimère et valence N-H amine secondaire), 3000 à 2800 (valence O-H acide type dimère et valence C-H aromatiques et alyphatiques), 1615 (valence C=O cétone conjuguée et valence asymétrique ion carboxylate O-C-O), 1484 (valence C=C aromatiques), 1331 (valence symétrique ion carboxylate O-C-O).

### 8-fluoropéfloxacine : Acide 1-éthyl-6,8-difluoro-1,4-dihydro-7-(4-méthyl-1-pipérazinyl)-4-oxoquinoline-3-carboxylique; RN = [75338-41-9].

**pF :** 235-8°C [litt. : 240-250°, EP 230.053 (1987)]
**RMN ¹H** (CDCl₃) en ppm : 1,57 (t; ³J = 7,4 Hz; 3H; CH₃ éthyle); 2,39 (s; 3H; CH₃ pipérazino); 2,5 à 2,65 (m; 4H; CH₂ pipérazino); 3,4 à 3,5 (m; 4H; CH₂ pipérazino); 4,4 à 4,6 (m; 2H; CH₂ éthyle); 7,93 (dd; ³J_{H-F6} = 11,8 Hz et ⁵J_{H-F8} = 1,4 Hz; 1H; H-5); 8,60 (s; 1H; H-2).
**RMN ¹³C** (CDCl₃) en ppm : 16,39 (d; ⁵J_{C-F8} = 5,4 Hz; CH₃ éthyle); 46,34 (s; CH₃ pipérazino); 50,87 (pseudo t; ⁴J_{C-F} ~ 4,3 Hz; 2 CH₂ pipérazino); 55,51 (s; 2 CH₂ pipérazino); 54,68 (d; ⁴J_{C-F8} = 16,8 Hz; CH2 éthyle); 108,25 (dd; ²J_{C-F6} = 22,9 Hz et ⁴J_{C-F8} = 3,0 Hz; CH-5); 121,08-121,27 (2 s; C-3 et C-8a); 127,31 (s; C-4a); 134,55 (t; ²J_{C-F6} ~ ²J_{C-F8} = 14,5 Hz; C-7); 145,88 (dd; ¹J_{C-F8} = 247,0 Hz et ³J_{C-F6} = 6,5 Hz; C-8); 150,06 (s; CH-2); 155,24 (dd; ¹J_{C-F6} = 250,1 Hz; ³J_{C-F8} = 6,5 Hz; C-6); 166,67 (s; COOH); 176,28 (s; CO).
**IR** (ATR) en cm⁻¹ : 3054 ( valence C-H aromatiques et aliphatiques), 3000 à 2800 (valence O-H acide type dimère et valence C-H aromatiques et aliphatiques), 1717 (valence C=O acide type dimère), 1619 (valence C=O cétone conjuguée et valence asymétrique ion carboxylate O-C-O), 1470 (valence C=C aromatiques), 1281 (valence C-F aromatique), 1243 (valence C-O acide type dimère), 926 (H-O déformation hors du plan).

### Acide 1-éthyl-6,8-difluoro-1,4-dihydro-7-(4-éthyl-1-pipérazinyl)-4-oxoquinoline-3-carboxylique; RN = [79668-41-0].

**pF** : 234-6°C. [litt. : 236-9°C, US 4,398,029 (1983)].
**RMN ¹H** (CDCl₃) en ppm : 1,15 (t; ³J = 7,2 Hz; 3H; CH₃ éthyle pipérazino); 1,57 (t; ³J = 6,8 Hz; 3H; CH₃ éthyle quinolino); 2,52 (q; ³J = 6,8 Hz; 2H; CH₂ éthyle pipérazino); 2,55 à 2,65 (m; 4H; 2 CH₂ cycle pipérazino); 3,4 à 3,55 (m; 4H; 2 CH₂ cycle pipérazino); 4,4 à 4,6 (m; 2H; CH₂ éthyle quinolino); 7,95 (dd; ³J_{H-F6} = 11,8 Hz et ⁵J_{H-F8} = 2,0 Hz; 1 H; H-5); 8,60 (s; 1 H; H-2).
**RMN ¹³C** (CDCl₃) en ppm : 11,88 (s; CH₃ éthyle pipérazino); 16,35 (d; ⁵J_{C-F8} = 4,6 Hz; CH₃ éthyle quinolino); 50,88 (pseudo t; ⁴J_{C-F} ~ 4,3 Hz; 2 CH₂ cycle pipérazino); 52,48 (s; CH₂ éthyle pipérazino); 53,23 (s; 2 CH₂ pipérazino); 54,67 (d; ⁴J_{C-F8} = 16,8 Hz; CH₂ éthyle quinolino); 108,13 (dd; ²J_{C-F6} = 22,9 Hz et ²J_{C-F8} = 3,4 Hz; CH-5); 120,89 - 121,08 (2 s; C-3 et C-8a); 127,21 (d; ³J_{C-F6} = 7,5 Hz; C-4a); 134,50 (t; ²J_{C-F6} ~ ²J_{C-F8} = 13,8 Hz; C-7); 145,78 (dd; ¹J_{C-F8} = 247,4 Hz et ³J_{C-F6} = 6,1 Hz; C-8); 150,01 (s; CH-2); 155,01 (dd; ¹J_{C-F6} = 244,7 Hz et ³J_{C-F8} = 6,5 Hz; C-6); 166,64 (s; COOH); 176,19 (s; CO).
**IR** (ATR) en cm⁻¹ : 3055 (valence C-H aromatiques et aliphatiques), 3000 à 2700 (valence O-H acide type dimère et valence C-H aromatiques et aliphatiques), 1716 (valence C=O acide type dimère), 1620 (valence C=O cétone conjuguée et valence asymétrique ion carboxylate O-C-O), 1470 (valence C=C aromatiques); 1286 (valence C-F aromatique), 1241 (valence C-O acide type dimère), 926 (H-O déformation hors du plan).

### Acide 1-éthyl-6,8-difluoro-1,4-dihydro-7-morpholinyl-4-oxoquinoline-3-carboxylique; RN = [79660-59-6].

**pF :** 260-270°C [litt. : 268-271°C, décomp.; US 4,398,0 29 (1983)].
**RMN ¹H** (5 % NaOD /D₂O) en ppm : 1,40 (t; ³J = 6,7 Hz; 3H; CH₃ éthyle); 3,20 à 3,40 (m; 4H; CH₂ morpholino); 3,8 à 3,95 (m; 4H; CH₂ morpholino); 4,20 à 4,40 (m; 2H; CH₂ éthyle); 7,54 (d; ³J_{H-F} = 12,8 Hz; 1 H; H-5); 8,33 (s; 1 H; H-2).
**RMN ¹³C** (5 % NaOD /D₂O) en ppm : 17,91 (d; ⁵J_{C-F8} = 4,6 Hz; CH₃ éthyle); 53,39 (s; 2 CH₂N morpholino); 56,17 (d; ⁴J_{C-F8} = 16,1 Hz; CH₂ éthyle); 69,70 (s; 2 CH₂O morpholino); 109,67 (d; ²J_{C-F6} = 22,9 Hz; CH-5); 119,01 (s; C-3); 126,18 (d; ²J_{C-F8} = 7,7 Hz; C-8a); 129,19 (d; ³J_{C-F6} = 7,6 Hz; C-4a); 134,46 (pseudo t; ²J_{C-F6} et ²J_{C-F8} = 13,0 et 14,5 Hz; C-7); 148,73 (dd; ¹J_{C-F8} = 249,0 Hz et ³J_{C-F6} = 6,9 Hz; C-8); 152,26 (s; CH-2); 156,82 (dd; ¹J_{C-F6} = 246,7 Hz et ³J_{C-F8} = 6,0 Hz; C-6); 174,53 (s; CO₂H); 176,65 (s; CO).
**IR** (ATR) en cm⁻¹ : 3054 (valence C-H aromatiques et aliphatiques, 3000 à 2800 (valence O-H acide type dimère et valence C-H aromatiques et aliphatiques), 1712 (valence C=O acide type dimère), 1619 (valence C=O cétone conjuguée et valence asymétrique ion carboxylate O-C-O), 1470 (valence C=C aromatiques), 1280 (valence C-F aromatique), 1239 (valence C-O acide type dimère), 921 (H-O déformation hors du plan)

### 8-fluoronorfloxacine : Acide 1-éthyl-6,8-difluoro-1,4-dihydro-7-(pipérazinyl)-4-oxoquinoline-3-carboxylique; RN = [99726-76-8].

**pF :** 253-4°C. [litt. : 232-6°C; WO 8,810,253 (1988)].
**RMN ¹H** (5 % NaOD /D₂O) en ppm : 1,39 (t; ³J = 6,6 Hz; 3H; CH₃ éthyle); 2,85 à 3,05 (m; 4H; 2 CH₂ cycle pipérazino); 3,15 à 3,35 (m; 4H; 2 CH₂ cycle pipérazino); 4,2 à 4,45 (m; 2H; CH₂ éthyle); 7,58 (d; ³J_{H-F} = 12,4 Hz; H-5); 8,32 (s; 1 H; H-2).
**RMN ¹³C** (5 % NaOD /D₂O) en ppm : 18,06 (d; ⁵J_{C-F8} = 4,6 Hz; CH₃ éthyle); 47,75 - 53,95 (2 s; CH₂ cycle pipérazino); 56,22 (d; 4J_{C-F8} = 16,7 Hz; CH₂ éthyle); 109,73 (d; ²J_{C-F6} = 22,9 Hz; CH-5); 118,77 (s; C-3); 125,78 (d; ²J_{C-F8} = 7,7 Hz; C-8a); 129,09 (d; ³J_{C-F6} = 6,9 Hz; C-4a); 135,11 (t; ²J _{C-F6} ~ ²J _{C-F8} = 13,8 Hz; C-7); 148,35 (dd; ¹J_{C-F8} = 248,2 Hz et ³J_{C-F6} = 5,7 Hz; C-8); 152,52 (s; CH-2); 156,62 (dd; ¹J_{C-F6} = 246,6 Hz et ³J_{C-F8} = 5,7 Hz; C-6); 174,40 (s; COOH); 176,62 (s; CO).
**IR** (ATR) en cm⁻¹ : 3600 à 3200 (valence O-H lié type dimère et valence N-H amine secondaire), 3100 à 2800 et 2800 à 2200 (valence O-H acide type dimère et valence C-H aromatiques ou aliphatiques), 3033 (valence C-H aromatiques), 1634 (valence C=O acide type dimère), 1616 (valence C=O cétone conjuguée et valence asymétrique ion carboxylate O-C-O), 1473 (valence C=C aromatiques), 1279 (valence C-F aromatique), 944 (H-O déformation hors du plan).

### Sparfloxacine : Acide 5-amino-1-cyclopropyl-7-[(3R,5S)-3,5-diméthyl-1-pipérazinyl]-6,8-difluoro-1,4-dihydro-4-oxoquinoline-3-carboxylique (stéréochimie relative); RN (x HCl) = [127585-83-5].

**pF :** > 285°C (x HCl).[litt : 266-9°C (base); Merck Ind ex 14th, 8735]
**RMN ¹H** (5 % NaOD /D₂O) en ppm : 0,85 à 1,0 (m; 2H; CH₂ cyclopropyle); 1,04 (d; ³J = 6,3 Hz; 6H; 2 CH₃ pipérazino), 1,0 à 1,20 (m; 2H; CH₂ cyclo-propyle); 2,65 à 3,0 (2 m; 4H; 2 CH et 1 CH₂ pipérazino), 3,1 à 3,3 (d large; 2H; CH₂ pipérazino), 3,65 à 3,8 (m; 1 H; CH cyclopropyle); 8,27 (s; 1 H; H-2). **RMN ¹³C** (5 % NaOD /D₂O) en ppm : 11,0 (s; CH₂ cyclopropyle); 20,6 (s; 2 CH₃ pipérazino); 41,89 (d; ⁴J_{C-F8} = 15,0 Hz; CH cyclopropyle); 53,14 (s; 2 CH pipérazino); 59,54 (s; 2 CH₂ pipérazino), 111,49 (d; ³J_{C-F6} = 4,6 Hz; C-4a); 117,99 (s; C-3); 130,62 (d; ²J_{C-F6} = 6,0 Hz; C-5); 134,81 (pseudo t; ²J_{C-F} = 11,5 et 12,95 Hz; C-7); 137,47 (d, ²J_{C-F8} = 12,3 Hz; C-8a); 138,82 (dd; ¹J_{C-F8} = 235,9 Hz et ³J_{C-F6} = 5,5 Hz; C-8); 142,20 (dd; ¹J_{C-F6} = 234,4 Hz et ³J_{C-F8} = 5,5 Hz; C-6); 151,19 (s; CH-2); 174,51 (s; CO₂H); 181,64 (s; CO).
**IR** (ATR) en cm⁻¹ : 3412 (valence O-H lié type dimère et valence N-H amine secondaire), 3272 (valence N-H primaire lié), 3200 à 2800 et 2800 à 2200 (valence O-H acide type dimère et valence C-H aromatiques ou aliphatiques), 1711 (valence C=O acide type dimère), 1632 (valence C=O cétone conjuguée), 1514 (valence asymétrique ion carboxylate O-C-O et vibration de déformation de N-H primaire), 1435 (valence C=C aromatiques), 1295 (valence C-F aromatique).

### Acide 1-éthyl-6,8-difluoro-1,4-dihydro-7-(4-(2-méthoxyphényl)-1-pipérazinyl)-4-oxoquinoline-3-carboxylique; RN = [153468-44-1]

**pF :** 217-8°C, décomposition.
**RMN ¹H** (CDCl₃) en ppm : 1,58 (t; ³J = 6,9 Hz; 3H; CH₃ éthyle); 3,1 à 3,35 (m; 4H; 2 CH₂ cycle pipérazino); 3,5 à 3,7 (m; 4H; 2 CH₂ cycle pipérazino); 3,91 (s; 3H; CH₃ méthoxy); 4,4 à 4,6 (m; 2H; CH₂ éthyle); 6,8 à 7,1 (m; 4H; H aromatiques); 7,93 (dd; ³J_{H-F} = 11,8 Hz et ⁵J_{H-F} = 1,4 Hz; 1 H; H-5); 8,61 (s; 1 H; H-8); 14,7 (s large; COOH).
**RMN ¹³C** (CDCl₃) en ppm : 16,37 (d; ⁵J_{C-F8} = 4,6 Hz; CH₃ éthyle); 51,26 - 51,35 (2 s; 4 CH₂ cycle pipérazino); 54,68 (d; ⁴J_{C-F8} = 16,8 Hz; CH₂ éthyle); 55,48 (s; CH₃ méthoxy); 107,91 (s; C-3); 108,20 (dd; ²J_{C-F6} = 23,7 Hz et ⁴J_{C-F8} = 3,1 Hz; CH-5); 111,38 - 118,29 - 121,06 - 123,41 (4 s; 4 CH du noyau 2-méthoxyphényle); 121,23 (s; C-8a); 127,21 (d; ³J_{C-F6} = 6,9 Hz; C-4a); 134,58 (pseudo t; ²J_{C-F} = 13,0 Hz et ²J_{C-F} = 14,6 Hz; C-7); 141,04 (s; C-1'); 145,92 (dd; ¹J_{C-F8} = 247,4 Hz et ³J_{C-F6} = 6,5 Hz; C-8); 150,11 (s; CH-2); 152,30 (s; C-2'); 155,18 (dd; ¹J_{C-F6} = 250,5 Hz et ³J_{C-F8} = 6,5 Hz; C-6); 166,63 (s; CO₂H); 176,22 (CO).
**IR** (ATR) en cm⁻¹ : 3055 (valence C-H aromatiques et aliphatiques), 3000 à 2750 (valence O-H acide type dimère et valence C-H aromatiques et aliphatiques), 1718 (valence C=O acide type dimère), 1622 (valence C=O cétone conjuguée et valence asymétrique ion carboxylate O-C-O), 1473 (valence C=C aromatiques), 1282 (valence C-F aromatique), 1239 (valence C-O acide type dimère), 927 (H-O déformation hors du plan)

### Acide 1-éthyl-6,8-difluoro-1,4-dihydro-7-(4-hydroxypipéridino)-4-oxoquinoline-3-carboxylique ; RN = [79666-56-3].

**pF**: 211°C [litt. : 216-8°C; US 4,398,029 (1983)].
**RMN ¹H** (5 % NaOD /D₂O) en ppm : 1,39 (t large; ³J = 7,0 Hz; 3H; CH₃ éthyle); 1,5 à 1,8 (m; 2H; CH₂ pipéridino); 1,90 à 2,1(m; 2H; CH₂ pipéridino); 3,05 à 3,15 (m; 2H; CH₂ pipéridino); 3,3 à 3,5 (m; 2H; CH₂ pipéridino); 3,85 (m; 1 H; CH pipéridino); 4,31 (m; 2H; CH₂ éthyle); 7,60 (d; ³J_{H-F} = 12,2 Hz; 1H; H-5); 8,31 (s; 1H; H-2).
**RMN** ¹³C (5 % NaOD /D₂O) en ppm : 18,07 (d; ⁵J_{C-F8} = 5,0 Hz; CH₃ éthyle); 36,68 (s; 2 CH₂ pipéridino); 51,66 (s; CH₂-N pipéridino); 56,30 (d; ⁴J_{C-F8} = 16,8 Hz; CH₂ éthyle); 57,05 (s; CH₂-N pipéridino); 69,97 (s; CH pipéridino); 109,76 (d; ²J_{C-F6} = 22,2 Hz; CH-5); 119,02 (s; C-3); 125,89 (d; ³J_{C-F6} = 6,9 Hz; C-4a); 129,32 (d; ²J_{C-F8} = 7,7 Hz; C-8a); 135,46 (pseudo t; ²J_{C-F6} ~ ²J_{C-F8} = 13,7 Hz; C-7); 148,72 (dd; ¹J_{C-F8} = 248,5 Hz; ³J_{C-F6} = 6,5 Hz; C-8); 152,40 (s; CH-2); 156,99 (dd; ¹J_{C-F6} = 244,5 Hz et ³J_{C-F8} = 6,0 Hz; C-6); 174,66 (s; COOH); 176,83 (s; CO).
**IR** (ATR) en cm⁻¹ : 3500 à 3150 (valence O-H lié type dimère), 3050 (valence C-H aromatiques et aliphatiques), 3000 à 2800 et 2800 à 2200 (valence O-H acide type dimère et valence C-H aromatiques et aliphatiques), 1716 (valence C=O acide type dimère), 1620 (valence C=O cétone conjuguée et valence asymétrique ion carboxylate O-C-O), 1464 (valence C=C aromatiques), 1280 (valence C-F aromatique), 926 (H-O déformation hors du plan).

### Flérofloxacine : Acide 1-(2-fluoro-1-éthyl)-6,8-difluoro-1,4-dihydro-7-(4-méthyl-1-pipérazinyl)-4-oxoquinoline-3-carboxylique; RN = [79660-72-3].

**pF :** 271-3°C
**RMN ¹H** (5 % NaOD /D₂O) en ppm : 2,30 (s; 3H; CH₃ pipérazino); 2,4 à 2,75 (m; 4H; 2 CH₂ pipérazino); 3,2 à 3,5 (m; 4H; 2 CH₂ pipérazino); 4,5 à 5,1 (m; 4H; 2 CH₂ fluoroéthyle); 7,68 (d; ³J_{H-F} = 12,6 Hz; 1H; H-5); 8,29 (s; 1H; H-2).
**RMN ¹³C** (5 % NaOD /D₂O) en ppm : 42,73 (s; CH₃ pipérazino); 48,02 - 52,28 (2 s; 4 CH₂ pipérazino); 55,65 (d, ²J_{C-F} = 19,1 Hz; N1-CH₂); 80,52 (d; ¹J_{C-F} = 164,9 Hz; CH₂-F); 105,26 (d; ²J_{C-F6} = 24,5 Hz; CH-5); 114,41 (s; C-3); 121,24 (d; ²J_{C-F8} = 8,5 Hz; C-8a); 124,57 (d; ³J_{C-F6} = 7,5 Hz; C-4a); 130,29 (t; ²J_{C-F} = 14,0 Hz; C-7); 144,07 (dd; ¹J_{C-F8} = 246,0 Hz et ³J_{C-F6} = 6,0 Hz, C-8); 148,52 (s; CH-2); 152,13 (dd; ¹_{JC-F6} = 244,5 Hz et ³J_{C-F8} = 6,5 Hz; C-6); 169,67 (s; CO₂H); 172,28 (s; CO).
**IR** (ATR) en cm⁻¹ : 3050 (valence C-H aromatiques et aliphatiques), 3000 à 2700 (valence O-H acide type dimère et valence C-H aromatiques et aliphatiques), 1716 (valence C=O acide type dimère), 1625 (valence C=O cétone conjuguée et valence asymétrique ion carboxylate O-C-O), 1476 (valence C=C aromatiques), 1281 (valence C-F aromatique), 926 (H-O déformation hors du plan).

### Enofloxacine : Acide 1-éthyl-6-fluoro-1,4-dihydro-4-oxo-7-(1-pipérazinyl)-1,8-naphthyridine-3-carboxylique; RN = [74011-58-8].

**pF :** 220°C [litt.: 220-4°C; Merck index 14th, 3587]
**RMN ¹H** (DMSO-d6) en ppm : 1,42 (t; ³J = 7,0 Hz; 3H; CH₃ éthyle); 2,7 à 3,0 (m; 4H; 2 CH₂ pipérazino); 3,7 à 3,9 (m; 4H; 2 CH₂ pipérazino); 4,52 (q large; ³J = 7,0 Hz; 2H; CH₂ éthyle); 8,09 (d; ³J_{H-F} = 14,2 Hz; 1 H; H-5); 9,01 (s; 1 H; H-2).
**RMN ¹³C** (5 % NaOD /D₂O) en ppm : 16,78 (s; CH₃ éthyle); 47,17 (s; 2 CH₂ pipérazino); 49,66 - 49,98 - 50,13 (3 s; 2 CH₂ pipérazino et CH₂ éthyle); 117,33 (d; ⁴J_{C-F6} = 3,9 Hz; C-4a); 120,44 (s; C-3); 122,22 (d; ²J_{C-F6} = 21,4 Hz; CH-5); 146,84 (s; C-8a); 148,28 (s; CH-2); 149,48 (d; ¹J_{C-F6} = 256,6 Hz; C-6); 152,35 (d; ²J_{C-F} = 9,2 Hz; C-7); 175,12 (s; CO₂H); 178,22 (s; CO).
**IR** (ATR) en cm⁻¹ : 3050 (valence C-H aromatiques et aliphatiques), 3000 à 2800 (valence O-H acide type dimère et valence C-H aromatiques et aliphatiques), 1713 (valence C=O acide type dimère), 1623 (valence C=O cétone conjuguée et valence asymétrique ion carboxylate O-C-O), 1475 (valence C=C aromatiques), 1287 (valence C-F aromatique), 950 (H-O déformation hors du plan).

### Témafloxacine : Acide 1-(2,4-difluorophényl)-6-fluoro-1,4-dihydro-7-(3-méthyl-1-pipérazinyl)-4-oxoquinoline-3-carboxylique; RN = [108319-06-8].

**pF :** 125-130°C, décomposition.
**RMN ¹H** (5% NaOD /D₂O) en ppm : 0,99 (d; ³J = 6,2 Hz; 3H; CH₃ pipérazino); 2,24 (t; J = 11,1 Hz; 1 H; H de CH₂ pipérazino); 2,57 (t large; J = 10,6 Hz; 1 H; H de CH₂ pipérazino); 2,70 à 3,0 (m; 3H; H de CH₂ pipérazino); 3,2 à 3,4 (m; 2H; CH₂ pipérazino); 6,39 (d; ⁴J_{H-F} = 7,0 Hz; 1 H; H-8); 7,2 à 7,4 (m; 2H aromatiques); 7,55 à 7,7 (m; 1 H aromatique); 7,90 (d; ³J_{H-F} = 13,2 Hz; 1 H; H-5); 8,37 (s; 1H; H-2).
**RMN ¹³C** (5% NaOD /D₂O) en ppm : 16,00 (s; CH₃ pipérazino); 41,90 (s; 2 CH₂ pipérazino); 47,26 (s; CH pipérazino); 54,25 (s; CH₂ pipérazino); 103,56 (s; C-8); 109,65 (d; ²J_{C-F} = 22,5 Hz; CH-5 ou CH-3' aromatique); 111,18 (d; ²J_{C-F} = 22,5 Hz; CH-3' aromatique ou CH-5); 115,96 (s; C-3); 119,32 (d; ³J_{C-F6} = 7,6 Hz; C-4a); 121,83 (d; ²J_{C-F} = 13,0 Hz; C-1' aromatique); 128,3 - 128,42 (2 s; CH-5' et CH-6' aromatiques); 136,01 (s; C-8a); 142,61 (d; ²J_{C-F6} = 10,7 Hz; C-7); 145,02 (s; CH-2); 150,99 (d; ¹J_{C-F6} = 247,5 Hz; C-6); 155,29 (dd; ¹J_{C-F} = 252,5 Hz et ³J_{C-F} = 15,5 Hz; C-2' ou C-4' aromatique); 161,19 (dd; ¹J_{C-F} = 249,5 Hz et ³J _{C-F} = 12 Hz; C-2' ou C-4' aromatique); 169,83 (s; CO₂H); 173,84 (s; CO).
**IR** (ATR) en cm⁻¹ : 3600 à 3200 (valence O-H lié type dimère et valence N-H amine secondaire), 3020 (valence C-H aromatiques et aliphatiques), 3000 à 2800 et 2800 à 2200 (valence acide type dimère et valence C-H aromatiques et aliphatiques), 1626 (valence C=O cétone conjuguée et valence asymétrique ion carboxylate O-C-O), 1470 (valence C=C aromatiques), 1286 (valence C-F aromatique).

### Difloxacine : Acide 1-(4-fluorophényl)-6-fluoro-1,4-dihydro-7-(4-méthyl-1-pipérazinyl) -4-oxoquinoline-3-carboxylique; RN = [98106-17-3].

**pF :** 260-265°C.
**RMN ¹H** (CDCl₃) en ppm : 2,33 (s; 3H; CH₃ pipérazino); 2,5 à 2,6 (m; 4H; CH₂ pipérazino); 3,05 à 3,2 (m; 4H; CH₂ pipérazino); 6,8 (d; ⁴J_{H-F6} = 7,2 Hz; 1 H; H-8); 7,25 à 7,50 (m; 4H aromatiques); 8,05 (d; ³J_{H-F6} = 13,2 Hz; 1H; H-5); 8,64 (s; 1H; H-2); 14,9 (s large; CO₂H).
**RMN ¹³C** (CDCl₃), en ppm : 46,10 (s; CH₃ pipérazino); 49,56 (d; ⁴J_{C-F6} = 4,6 Hz; 2 CH₂ pipérazino); 54,65 (s; 2 CH₂ pipérazino); 105,62 (d; ⁴J_{C-F6} = 3,8 Hz; CH-8); 108,62 (s; C-3); 112,49 (d; ²J_{C-F6} = 23,7 Hz; CH-5); 117,98 (d; ²J_{C-F4'} = 23,6 Hz; CH-3' et CH-5' aromatiques); 119, 57 (s; C-4a); 129,20 (d; ³J_{C-F6} = 9,2 Hz; CH-2' et CH-6' aromatiques); 136,23 (d; ⁴J_{C-F4'} = 3,5 Hz; C-1' aromatique); 139,33 (s; C-8a); 146,01 (d; ²J_{C-F6} = 10,7 Hz; C-7); 147,76 (s; CH-2); 153,68 (d; ¹J_{C-F6} = 250,5 Hz; C-6); 163,36 (d; ¹J_{C-F4'} = 252,0 Hz; C-4' aromatique); 166,92 (s; CO₂H); 177,35 (s; CO)
**IR** (ATR) en cm⁻¹ : 3050 (valence C-H aromatiques et aliphatiques), 3000 à 2700 (valence O-H acide type dimère et valence C-H aromatiques et aliphatiques), 1733 (valence C=O acide type dimère), 1627 (valence C=O cétone conjuguée et valence asymétrique ion carboxylate O-C-O), 1490 (valence C=C aromatiques), 1294 (valence C-F aromatique).

### Sarafloxacine : Acide 1-(4-fluorophényl)-6-fluoro-1,4-dihydro-7-(1-pipérazinyl)-4-oxoquinoline-3-carboxylique; RN = [98105-99-8].

**pF :** 260-270°C.
**RMN ¹H** (5% NaOD /D₂O) en ppm : 2,80 à 3,1 (m; 8H; 4 CH₂ pipérazino); 6,46 (d; ³J_{H-F} = 7,0 Hz; 1H; H-8); 7,3 à 7,6 (m; 4H aromatiques); 7,84 (d; ⁴J_{H-F} = 13,8 Hz; 1H; H-5); 8,41 (s; 1H; H-2).
**RMN ¹³C** (5% NaOD /D₂O) en ppm : 42,04 - 48,18 (2 s; 4 CH₂ piperazino); 104,63 (s; CH-8); 109,37 (d; ²J_{C-F} = 23,7 Hz; CH-5); 115,19 (d; ²J_{C-F} = 23,7 Hz; 2 CH-3' et 5' aromatiques); 115,35 (s; C-3); 119,67 (d; ³J_{C-F} = 6,9 Hz; C-4a); 127,15 (d; ³J_{C-F} = 9,2 Hz; CH-2' et 6' aromatiques); 134,36 - 136,27 (2 s; C-1' aromatique et C-8a); 142,44 (d; ²J_{C-F} = 11,5 Hz; C-7); 144,82 (s; CH-2); 150,95 (d; ¹J_{C-F} = 246,6 Hz; C-6); 160,69 (d; ¹J_{C-F} = 246,7 Hz; C-4' aromatique); 170,17 (s; COOH); 173,39 (s; CO).
**IR** (ATR) en cm⁻¹ : 3650 à 3200 (valence O-H lié type dimère et valence N-H amine secondaire), 3070 (valence C-H aromatiques et aliphatiques), 3000 à 2800 et 2800 à 2200 (valence O-H acide type dimère et valence C-H aromatiques et aliphatiques), 1728 (valence C=O acide type dimère), 1619 (valence C=O cétone conjuguée et valence asymétrique ion carboxylate O-C-O), 1470 (valence C=C aromatiques), 1296 (valence C-F aromatique), 925 (H-O déformation hors du plan).

### Péfloxacine : Acide 1-éthyl-6-fluoro-1,4-dihydro-7-(4-méthyl-1-pipérazinyl)-4-oxoquinoline-3-carboxylique; RN = [70458-92-3].

**pF :** 266-272°C. [litt : 270-2°C. Merck Index Ed 14 ^{th}, 7066].
**RMN ¹H** (5% NaOD /D₂O), en ppm : 1,39 (t; ³J = 6,8 Hz; 3H; CH₃ éthyle); 2,33 (s; 3H; CH₃ pipérazino); 2,5 à 2,7 (m; 4H; 2 CH₂ pipérazino); 3,1 à 3,3 (m; 4H; 2 CH₂ pipérazino); 4,19 (pseudo q; 2H; CH₂ éthyle); 6,82 (d; ⁴J_{H-F} = 6,8 Hz; 1H; H-8); 7,71 (d; ³J_{H-F} = 13,8 Hz; 1 H; H-5); 8,36 (s; 1H; H-2).
**RMN ¹³C** (5 % NaOD /D₂O) en ppm : 16,29 (s; CH₃ éthyle); 47,23 (s; CH₃ pipérazino); 51,57 (s; CH₂ éthyle); 52,0 (d; ⁴J_{C-F6} = 3,8 Hz; 2 CH₂ pipérazino); 56,22 (s; 2 CH₂ pipérazino); 107,55 (s; CH-8); 114,28 (d; ²J_{C-F6} = 22,9 Hz; CH-5); 119,19 (s; C-3); 124,97 (d; ³J_{C-F6} = 9,5 Hz; C-4a); 138,88 (s; C-8a); 146,66 (d; ²J_{C-F6} = 11,4 Hz; C-7); 149,53 (s; CH-2); 155,30 (d; ¹J_{C-F6} = 245,9 Hz; C-6); 174,91 (s; CO₂H); 177, 66 (d; ⁴J_{C-F6} = 2,3 Hz; CO).
**IR** (ATR) en cm⁻¹ : 3060 (valence C-H aromatiques et aliphatiques), 3000 à 2750 (valence O-H acide type dimère et valence C-H aromatiques et aliphatiques), 1736 (valence C=O acide type dimère), 1625-1615 (valence C=O cétone conjuguée et valence asymétrique ion carboxylate O-C-O), 1475 (valence C=C aromatiques), 1256 (valence C-F aromatique), 950 (HO déformation hors du plan).

### Amifloxacine : Acide 1-méthylamino-6-fluoro-1,4-dihydro-7-(4-méthyl-1-pipérazinyl)-4-oxoquinoline-3-carboxylique; RN = [86393-37-5].

**pF :** 250-260°C.
**RMN ¹H** (5 % NaOD /D₂O) en ppm : 2,34 (s; 3H; CH₃ pipérazino); 2,55 à 2,85 (m; 4H; 2 CH₂ pipérazino); 2,87 (s; 3H; NCH₃), 3,1 à 3,3 (m; 4H; 2 CH₂ pipérazino); 7,34 (d; ⁴J_{H-F6} = 6,8 Hz; 1H; H-8); 7,70 (d; ³J_{H-F6} = 13,6 Hz; 1H; H-5); 8,53 (s; 1 H; H-2).
**RMN ¹³C** (5 % NaOD /D₂O) en ppm : 35,82 (s; NCH₃); 42,50 (s; CH₃ pipérazino); 47,34 - 51,47 (2 s; 4 CH₂ pipérazino); 102,51 (s; CH-8); 109,31 (d; ²J_{C-F6} = 23,7 Hz; CH-5); 114,79 (s; C-3); 119,6 (d; ³J_{C-F6} = 6,8 Hz; C-4a); 134,90 (s; C-8a); 142,24 (d; ²J_{C-F6} = 10,6 Hz; C-7); 143,97 (s; CH-2); 150,75 (d; ¹J_{C-F6} = 246,6 Hz; C-6); 169,82 (s; CO₂H); 172,6 (d; ⁴J_{C-F6} = 2,3 Hz; CO).
**IR** (ATR) en cm⁻¹ : 3298 (valence N-H amine secondaire hydrazine), 3050 (valence C-H aromatiques et aliphatiques), 3000 à 2700 (valence O-H acide type dimère et valence C-H aromatiques et aliphatiques), 1718 (valence C=O acide type dimère), 1628 - 1611 (valence C=O cétone conjuguée et valence asymétrique ion carboxylate O-C-O), 1485 (valence C=C aromatiques), 1290 (valence C-F aromatique), 965 (H-O déformation hors du plan).

### Enrofloxacine : Acide 1-cyclopropyl-6-fluoro-1,4-dihydro-7-(4-éthyl-1-pipérazinyl)-4-oxoquinoline-3-carboxylique; RN = [93106-60-6].

**pF :** 222-4°C (litt : 219-221°C, Merck Index 14th, 3592 ).
**RMN ¹H** (CDCl₃) en ppm : 1,16 (t; ³J = 7,4 Hz; 3H; CH₃ éthyle); 1,16 à 1,24 (m; 2H; CH₂ cyclopropyle); 1,30 à 1,45 (m; 2H; CH₂ cyclopropyle); 2,52 (q; ³J = 6,8 Hz; 2H; CH₂ éthyle); 2,65 à 2,75 (m; 4H; 2 CH₂ pipérazino), 3,35 à 3,42 (m; 4H; 2 CH₂ pipérazino), 3,56 (m; 1H; CH cyclopropyle); 7,35 (d; ⁴J_{H-F6} = 7,4 Hz; 1H; H-8); 7,99 (d; ³J_{H-F6} = 13,2 Hz; 1H; H-5); 8,75 (s; 1H; H-2).
**RMN ¹³C** (CDCl₃), en ppm : 8,28 (s; 2 CH₂ cyclopropyle); 12,03 (s; CH₃ éthyle); 35,41 (s; CH cyclopropyle); 49,79 - 49,80 - 52,34 - 52,51 (4 s; 4 CH₂ pipérazino); 104,88 (d; ³J_{C-F6} = 3,8 Hz; CH-8); 108,01 (s; C-3); 112,26 (d; ²J_{C-F6} = 23,7 Hz; CH-5); 119,63 (d; ³J_{C-F6} = 8,4 Hz; C-4a); 139,17 (s; C-8a); 146,04 (d; ²J_{C-F6} = 10,7 Hz; C-7); 147,42 (s; CH-2); 153,73 (d, ¹J_{C-F6} = 250,4 Hz; C-6); 167,13 (s; CO₂H); 177,09 (d; ⁴J_{C-F6} = 3,0 Hz; CO).
**IR** (ATR) en cm⁻¹ : 3500 à 3100 (valence O-H lié type dimère), 3080 (valence C-H aromatiques et aliphatiques), 3000 à 2700 (valence O-H acide type dimère et valence C-H aromatiques et aliphatiques), 1735 (valence C=O acide type dimère), 1626 (valence C=O cétone conjuguée et valence asymétrique ion carboxylate O-C-O), 1464 (valence C=C aromatiques), 1291 (valence C-F aromatique), 955 (H-O déformation hors du plan).

### Ciprofloxacine : Acide 1-cyclopropyl-6-fluoro-1,4-dihydro-7-(1-pipérazinyl)-4-oxoquinoline-3-carboxylique; RN = [85721-33-1].

**pF :** 260-5°C [litt : 255-7°C, décomposition; Merck Ind ex 14th, 2314].
**RMN ¹H** (5 % NaOD / D₂O) en ppm : 0,95 à 1,1 (m; 2H; CH₂ cyclopropyle); 1,2 à 1,4 (m; 2H; CH₂ cyclopropyle); 2,9 à 3,15 (m; 4H; 2 CH₂ pipérazino); 3,15 à 3,25 (m; 4H; 2 CH₂ pipérazino); 3,4 à 3,6 (m; 1H; CH cyclopropyle), 7,39 (d; ⁴J_{H-F} = 7,0 Hz; 1H; H-8); 7,69 (d; ³J_{H-F} = 13,6 Hz; 1 H; H-5); 8,45 (s; 1H; H-2).
**RMN ¹³C** (5 % NaOD / D₂O) en ppm : 5,32 (s; 2 CH₂ cyclopropyle); 32,63 (s; CH cyclopropyle); 42,19 (s; 2 CH₂ pipérazino); 48,55 (d; ⁴J_{C-F6} = 3,8 Hz; 2 CH₂ pipérazino), 104,19 (s; CH-8); 109,27 (d; ²J_{C-F6} = 23,6 Hz; CH-5); 114,27 (s; C-3); 119,84 (d; ²J_{C-F6} = 7,6 Hz; C-4a); 136,27 (s; C-8a); 142,36 (d; ²J_{C-F6} = 10,7 Hz; C-7); 144,98 (s; CH-2); 151,02 (d; ¹J_{C-F6} = 245,9 Hz; C-6); 170,36 (s; CO₂H); 173,38 (s; CO).
**IR** (ATR) en cm⁻¹ : 3600 à 3200 (valence O-H lié type dimère et valence N-H amine secondaire), 3100 à 2500 (valence acide O-H type dimère et valence C-H aromatiques et aliphatiques), 1615 (valence C=O cétone conjuguée et valence asymétrique ion carboxylate O-C-O), 1478 (valence C=C aromatiques), 1291 (valence C-F aromatique), 941 (H-O déformation hors du plan).

### Acide 1-cyclopropyl-6-fluoro-1,4-dihydro-4-oxo-7-(1-pipérazinyl)-1,8-naphthyridin-3-carboxylique; RN = [99735-41-8].

**pF :** 266°C [Litt. : 274-6°C; EP153163 (1985)]
**RMN ¹H** (5 % NaOD / D₂O) en ppm : 1,1 à 1,3 (m; 4H; 2 CH₂ cyclo-propyle); 2,8 à 3,0 (m; 4H; 2 CH₂ pipérazino); 3,38 (m; 1H; CH cyclo-propyle); 3,6 à 3,8 (m; 4H; 2 CH₂ pipérazino); 7,69 (d; ³J = 14,2 Hz; 1 H; H-5); 8,36 (s; 1 H; H-2).
**RMN ¹³C** (5 % NaOD / D₂O) en ppm : 9,28 (s; 2 CH₂ cyclopropyle); 36,36 (s; CH cyclopropyle), 47,20 (s; 2 CH₂ pipérazino); 49,90 (d; ⁴J_{C-F6} = 7,7 Hz; 2 CH₂ pipérazino); 116,65 (s; C-3 ou C-4a); 119,47 (s; C-4a ou C-3); 122,16 (d; ²J_{C-F6} = 21,4 Hz; CH-5); 148,27 (s; C-7); 148,49 (s; CH-2); 148,99 (d; ¹J_{C-F6} = 250,5 Hz; C-6); 151,63 (s; C-8a); 174,51 (s; CO₂H); 178,09 (s; CO).
**IR** (ATR) en cm⁻¹ : 1723 (valence C=O acide type dimère), 1629 (valence C=O cétone conjuguée et valence asymétrique ion carboxylate O-C-O), 1438 (valence C=C aromatiques), 1262 (valence C-F aromatique), 952 (HO déformation hors du plan).

### Acide 1-(2,4-difluorophényl)-6-fluoro-1,4-dihydro-4-oxo-7-(1-pipérazinyl)-1,8-Naphthyridine-3-carboxylique; RN = [100490-19-5].

**pF :** 231°C [litt.: 274-6°C; EP153163 (1985).
**RMN ¹H** (5 % NaOD / D₂O) en ppm : 2,5 à 2,8 (m; 4H; 2 CH₂ pipérazino), 3,2 à 3,6 (m; 4H; 2 CH₂ pipérazino); 7,05 à 7,3 (m; 2H; 2 CH du noyau phényle); 7,3 à 7,5 (m; 1H; CH du noyau phényle); 7,82 (d; ³J = 13,6 Hz; 1 H; H-5); 8,38 (s; 1 H; H-2).
**RMN ¹³C** (DMSO-d6) en ppm : 45,31 (s; 2 CH₂ pipérazino); 48,03 (d; ⁴J_{C-F6} = 6,9 Hz, 2 CH₂ pipérazino); 104,75 (dd; ²J_{C-F} = 26,7 Hz et 27,5 Hz; CH-3'); 109,14 (s; C-3); 111,61 (d; ³J_{C-F6} = 3,1 Hz; C-4a); 112,25 (dd; ²J_{C-F4'} = 22,9 Hz et ⁴J_{C-F2'} = 3,1 Hz; CH-5' aromatique); 119,32 (d; ²J_{C-F6} = 22,9 Hz; CH-5); 124,03 (dd; ²J_{C-F2'} = 13,0 Hz et ⁴J_{C-F4'} = 3,8 Hz; C-1' aromatique); 130,87 (d; ³J_{C-F} = 10,7 Hz; CH-6' aromatique); 145,71 (s; C-8a); 146,89 (d; ¹J_{C-F6} = 258,1 Hz; C-6); 148,03 (s; CH-2); 149,74 (d; ²J_{C-F6} = 9,2 Hz; C-7); 157,26 (dd; ¹J_{C-F}= 251,2 Hz et ³J_{C-F} = 13,8 Hz; C-2' ou C-4' aromatique); 162,58 (dd; ¹J_{C-F} = 248,2 Hz et ³J_{C-F} = 11,8 Hz; C-4' ou C-2' aromatique); 165,36 (s; CO₂H); 176,89 (s; CO).
**IR** (ATR) en cm⁻¹ : 3600 à 3200 (valence O-H lié type dimère et valence N-H amine secondaire), 3100 à 2200 (valence O-H acide type dimère et valence C-H aromatiques et aliphatiques), 1626 (valence C=O cétone conjuguée), 1509 (valence asymétrique ion carboxylate O-C-O et vibration de déformation de N-H), 1442 (valence C=C aromatiques), 1269 (valence C-F aromatique).

Dans le cas de la condensation de certaines amines primaires avec les composés de type II, le procédé de préparation peut être modifié selon le protocole qui a été utilisé et est décrit ci-dessous.

### Exemple 2: Procédé de préparation de l'acide 1-éthyl-6,8-difluoro-1,4-dihydro-7-(benzylamino)-4-oxoquinoline-3-carboxylique.

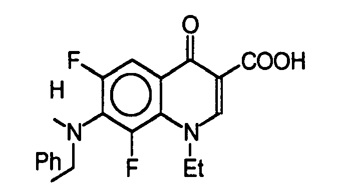

Dans un erlen de 50 ml, mettre en suspension 3 g de 1-éthyl-6,7,8-trifluoro-1,4-dihydro-4-oxoquinoline-3-carboxylate d'éthyle et 3,1 g de carbonate de potassium dans 18 ml d'eau permutée. Chauffer le milieu réactionnel à 90-95°C en masse pendant 24 heures jusqu'à dissolution totale puis ajouter 3,2 g de benzylamine et maintenir le chauffage pendant 8 heures supplémentaires en contrôlant l'évolution de la réaction par CCM. Lorsque la réaction est terminée, refroidir le milieu réactionnel à température ambiante, ajuster le pH à environ 7,5 par addition d'acide chlorhydrique à 15 %. Filtrer le précipité formé sur fritté et rincer successivement avec 6 ml d'eau permutée puis avec 6 ml d'acétone. Le produit obtenu est purifié par un aller et retour acide-base puis séché en étuve à 50°C. On obtient 2,2 g de cristaux blancs. (Rdt ~ 56 %).

### Acide 1-éthyl-6,8-difluoro-1,4-dihydro-7-(benzylamino)-4-oxoquinoline-3-carboxylique ; C₁₉H₁₆F₂N₂O₃ = 358,34.

**pF :** 200°C.
**RMN ¹H** (CDCl₃) en ppm : 1,50 (t; ³J = 6,8 Hz; 3H; CH₃ éthyle); 4,35 à 4,6 (m; 2H; CH₂ éthyle); 4,73 (s; 2H; CH₂ benzylique); 7,36 (s; 5 H du noyau phényle); 7,94 (dd; ³J_{H-F} = 12,0 Hz et ⁵J_{H-F} = 2,0 Hz; 1 H; H-5); 8.55 (s; 1 H; H-2); 14,9 (s large; CO₂H).
**RMN ¹³C** (5 % NaOD / D₂O) en ppm : 17,90 (s; CH₃ éthyle); 51,17 (s; CH₂ benzylique); 55,55 (s large; CH₂ éthyle); 109,41 (d; ²J_{C-F6} ~ 20,5 Hz; C-5); 117,92 (s; C-3); 121,36 (d; ²J_{C-F8} ~ 6,9 Hz; C-8a); 128,42 (d; ³J_{C-F6} ~ 5,0 Hz; C-4a); 129,51 - 129,84 - 130,81 (3 s; 5 CH du noyau phényle); 132,47 (pseudo t; ²J_{C-F} = 13,8 et 14,5 Hz; C-7); 141,93 (dd; ¹J_{C-F8} ~ 240 Hz et ³J_{C-F6} ~ 6,5 Hz; C-8); 142,30 (s; C-1' noyau phényle); 151,98 (dd; ¹J_{C-F6} ~ 241,0 Hz et ³J_{C-F8} ~ 8,0 Hz; C-6); 152,15 (s; CH-2); 174,27 (s; CO₂H); 176,63 (s; CO).
**IR** (ATR) en cm⁻¹ : 3600 à 3200 (valence O-H lié type dimère et valence N-H amine secondaire), 3160 à 2850 (valence acide type dimère et valence C-H aromatiques et aliphatiques), 1708 (valence C=O acide type dimère), 1633 et 1613 (valence C=O cétone conjuguée et valence asymétrique ion carboxylate O-C-O), 1484 (valence C=C aromatiques).

A titre d'exemples, les composés suivants sont préparés selon le mode opératoire de l'exemple 2 :

### Chlorhydrate de l'acide 1-éthyl-6,8-difluoro-1,4-dihydro-7-(phénéthylamino)-4-oxoquinoline-3-carboxylique; C₂₀H₁₈F₂N₂O₃; HCl = 408,83

**pF :** 160,5°C
**RMN ¹H** (CDCl₃) en ppm : 1,54 (dt; J = 7,0 et 1,2 Hz; 3H; CH₃ éthyle); 2,96 (t; ³J = 7 Hz; 2H; CH₂ phénéthyle); 3,83 (tt; J = 7,0 et 2,0 Hz; 2H; CH₂ phénéthyle); 4,43 (dq; J = 7,0 et 3,1 Hz; 2H, CH₂ éthyle); 7,15 à 7,35 (m; 5H aromatiques); 7,92 (dd; ³J_{H-F} = 12,2 Hz et ⁵J_{H-F} = 1,9 Hz; 1H; H-5); 8.56 (s; 1H; H-2).
**RMN ¹³C** (DMSO-d6) en ppm : 15.84 (d; ⁵J_{C-F8} = 5,4 Hz; CH₃ éthyle); 36,79 - 46,12 (2 s; 2 CH₂ phénéthyle); 53,44 (d; ⁴J_{C-F8} = 16,0 Hz; CH₂ éthyle); 106,37 (s; C-3); 106,50 (d; ²J_{C-F6} = 22,1 Hz; CH-5); 114,33 (d; J_{C-F} = 7,6 Hz; C-8a); 126,21 (s; CH-4' noyau phényle); 126,87 (d; J_{C-F} = 5,4 Hz; C-4a); 128,36 - 128,70 (2 s; 4 CH-3' et 5' noyau phényle); 132,27 (pseudo t; ²J_{C-F} = 13,7 et 14,6 Hz; C-7); 138,90 (s; C-1' noyau phényle); 139,04 (dd; ¹J_{C-F8} = 240,5 Hz et ³J_{C-F6} = 7,6 Hz; C-8); 150,27 (s; CH-2); 149,83 (dd; ¹J_{C-F6} = 244,4 Hz et ³ J_{C-F8} = 7,7 Hz; C-6); 165,82 (s; COOH); 175,26 (s; CO).
**IR** (cm-1): 3341 (valence O-H lié type dimère), 3200 à 2800 (valence acide O-H type dimère et valence C-H aromatiques et aliphatiques), 1717 (valence C=O acide type dimère), 1632 - 1614 (valence C=O cétone conjuguée et valence asymétrique ion carboxylate O-C-O), 1463 (valence C=C aromatiques), 1274 (valence C-F aromatique).

### Exemple 3 :

Les composés suivants ont été préparés selon le mode opératoire de l'exemple 1. Acide 6,8-difluoro-1,4-dihydro-1-(2-hydroxyéthyl)-7-(4-méthyl-1-pipérazinyl)-4-oxoquinoline -3-carboxylique ;
**pF :** > 300 °C.
**RMN ¹H** (DMSO-d₆) en ppm : 2,78 (s; 3H; CH₃ pipérazino); 3,0 à 3,5 (m; 4H; 2 CH₂ pipérazino); 3,5 à 3,7 (m; 4H; 2 CH₂ pipérazino); 3,79 (m; 2H; CH₂-N hydroxyéthyle); 4,64 (m; 2H; CH₂ hydroxyéthyle); 5,13 (s large; 1H; OH); 7,89 (d; ³J_{H-F6} = 11,8 Hz; 1 H; H-5); 8,76 (s; 1 H; H-2); 11,6 (s large; 1 H; COOH).
**RMN ¹³C** (DMSO-d₆) en ppm : 42,41 (s; CH₃ pipérazino); 47,40 - 52,92 (2 s; CH₂ pipérazino et CH₂ hydroxyéthyle); 59,24 (s; CH₂ hydroxyéthyle); 60,66 (d; ⁴J_{C-F} = 13,8 Hz; CH₂ pipérazino); 106,35 (s; C-3); 107,07 (d; ²J_{C-F} = 23,7 Hz; CH-5); 121,48 (pseudo d; C-8a); 127,28 (pseudo d; C-4a); 132,49 (s; C-7); 146,55 (d; ¹J_{C-F8} ~ 244,0 Hz; C-8); 152,68 (s; CH-2); 154,44 (d; ¹J_{C-F6} ~ 254,3 Hz; C-6); 165,63 (s; COOH); 175,71 (s; CO).
**IR** (ATR) en cm⁻¹ : 3600 à 3000 (valence O-H lié type dimère), 3064 (valence C-H aromatiques et aliphatiques), 3000 à 2800 (valence C-H aromatiques et aliphatiques), 2700 à 2400 (valence acide O-H lié type dimère), 1732 (valence C=O acide type dimère), 1625 - 1604 (valence C=O cétone conjuguée et valence asymétrique ion carboxylate O-C-O), 1476 (valence C=C aromatiques), 1286 (valence C-F aromatique), 807 (valence C-H aromatique isolé).

### Acide 1-éthyl-6,8-difluoro-1,4-dihydro-7-(4-méthyl-1-pipéridinyl)-4-oxoquinoline-3-carboxylique;

**pF** : 234°C
**RMN ¹H** (CDCl₃) en ppm : 1,02 (d; ³J = 5,8 Hz; 3H; CH₃ pipéridinyle); 1,25 à 1,55 (m; 2H; CH₂ pipéridinyle); 1,57 (td; ³J = 7,0 Hz et ⁶J_{H-F8} = 0,8 Hz; 3H; CH₃ éthyle); 1,65 à 1,85 (pseudo d; J ~ 12,0 Hz; 2H; CH₂ pipéridinyle); 3,23 (pseudo t; J ~ 11,5 Hz; 2H; CH₂ pipéridinyle); 3,35 à 3,55 (pseudo d; J ~ 12,2 Hz; 2H; CH₂ pipéridinyle); 4,48 (dq; ³J = 7,0 Hz et ⁵J_{H-F8} = 3,2 Hz; 2H; CH₂ éthyle); 7,91 (dd; ³J_{H-F6} ~ 12,2 Hz et ⁵J_{H-F8} ~ 2 Hz ; 1H; H-5); 8,60 (s; 1H; H-2).
**RMN ¹³C** (CDCl₃) en ppm : 16,42 (d; ⁵J_{C-F8} = 5,4 Hz; CH₃ éthyle); 22,08 (s; CH₃ pipéridinyle); 30,62 (s; CH pipéridinyle); 34,86 (s; 2 CH₂ pipéridinyle); 51,72 (pseudo t; J ~ 4,0 Hz; 2 CH₂ pipéridinyle); 54,73 (d; 4J_{C-F8} = 16,8 Hz; CH₂ éthyle); 107,82 (s; C-3); 108,06 (dd; ²J_{C-F6} = 23,7 Hz et ⁴J _{C-F8} ~ 3,5 Hz ; CH-5); 120,50 (d; ²J_{C-F8} = 9,2 Hz; C-8a); 127,27 (d; ³J_{C-F6} = 6,9 Hz; C-4a); 135,46 (pseudo t; ²J_{C-F} ~ 13,8 Hz; C-7); 145,75 (dd; ¹J_{C-F8} ~ 246,6 Hz et ³J_{C-F6} ~ 1,5 Hz; C-8); 149,95 (s; CH-2); 155,33 (dd; ¹J_{C-F6} ~ 249,7 Hz et ³J_{C-F8} ~ 1,3 Hz; C-6); 166,84 (s; COOH); 176,28 (s; CO).
**IR** (ATR) en cm⁻¹ : 3053 (valence C-H aromatiques et aliphatiques), 3000 à 2600 (valence acide O-H lié type dimère et valence C-H aromatiques et aliphatiques), 1717 (valence C=O acide type dimère), 1620 (valence C=O cétone conjuguée et valence asymétrique ion carboxylate O-C-O), 1470 (valence C=C aromatiques), 1279 (valence C-F aromatique), 926 (H-O déformation hors du plan), 808 (valence C-H aromatique isolé).

### Acide 1-éthyl-6,8-difluoro-1,4-dihydro-7-(3,4-dihydro-2(1H)-isoquinolinyl)-4-oxo-quinoline-3-carboxylique ; RN = [138276-64-9]

**pF :** 226°C
**RMN ¹H** (CDCl₃) en ppm :1,58 (dt; ³J = 7,4 Hz et ⁶J_{H-F8} = 1,6 Hz; 3H; CH₃ éthyle); 3,07 (t; ³J = 5,8 Hz; 2H; CH₂ tétrahydroisoquinolinyle); 3,68 (t; ³J = 5,8 Hz; 2H; CH₂-N tétrahydroisoquinolinyle); 4,46 (dq; ³J = 7,4 Hz et ⁵J_{H-F8} = 3,0 Hz; 2H; CH₂ éthyle); 4,62 (s; 2H; CH₂-N tétrahydroisoquinolinyle); 7,05 à 7,15 (m; 1 H; H aromatique tétrahydroisoquinolinyle); 7,15 à 7,30 (m; 3H; 3 H aromatiques tétrahydroisoquinolinyle); 7,97 (dd; ³J_{H-F8} ~ 12,0 Hz et ⁵J_{H-F6} ~ 2,0 Hz ; 1 H; H-5); 8,61 (s; 1 H; H-2).
**RMN ¹³C** (CDCl₃) en ppm : 16,47 (d; ⁵J_{C-F8} = 5,3 Hz; CH₃ éthyle); 29,62 (s; CH₂ tétrahydroisoquinolinyle); 49,35 (pseudo t; ⁴J ~ 3,8 Hz; CH2-N tétrahydroisoquinolinyle); 52,67 (s; CH₂-N tétrahydroisoquinolinyle); 54,74 (d; ⁴J_{C-F8} = 16,8 Hz; CH₂ éthyle); 108,11 (s; C-3); 108,44 (dd; ⁴J_{C-F8} ~ 3,0 Hz et ²J_{C-F6} ~ 23,7 Hz; CH-5); 121,02 (d; ²J_{C-F8} ~ 8,0 Hz; C-8a); 126,10 - 126,24 - 126,71 (3 s; CH aromatiques tétrahydroisoquinolinyle); 127,31 (d; ³J_{C-F6} = 7,0 Hz; C-4a); 129,32 (s; CH aromatique tétra-hydroisoquinolinyle); 133,83 - 134,09 (2 s, 2 C quaternaires tétrahydroisoquinolinyle); 134,58 (pseudo t; ²J_{C-F} ~ 14,5 Hz; C-7); 145,77 (dd; ¹J_{C-F8} = 247,4 Hz et ³J_{C-F6} ~ 6,1 Hz; C-8); 150,11 (s; CH-2); 155,16 (dd; ¹J_{C-F6} = 249,7 Hz et ³J_{C-F8} ~ 6,5 Hz; C-6); 166,77 (s; COOH); 176,36 (s; CO).
**IR** (ATR) en cm⁻¹ : 3100 à 2700 (valence C-H aromatiques et aliphatiques), 2700 à 2400 (valence acide O-H lié type dimère), 1721 (valence C=O acide type dimère), 1623 (valence C=O cétone conjuguée et valence asymétrique ion carboxylate O-C-O), 1468 (valence C=C aromatiques), 1276 (valence C-F aromatique), 949 (H-O déformation hors du plan) ; 803 (valence C-H aromatique isolé).

### Acide 1-éthyl-6,8-difluoro-1,4-dihydro-7-(thiomorpholinyl)-4-oxoquinoline-3-carboxylique ; RN = [93509-84-3].

**pF :** 287 °C [litt. : > 300°C; US 4,473,568 (1984)]
**RMN ¹H** (CDCl₃) en ppm : 1,57 (dt; ³J = 7,2 Hz et ⁶J_{H-F8} ~ 1,0 Hz; 3H; CH₃ éthyle); 2,7 à 2,9 (m; 4H; CH₂ thiomorpholino); 3,55 à 3,7 (m; 4H; CH₂ thiomorpholino); 4,4 à 4,6 (dq; ³J = 7,2 Hz et ⁵J_{H-F8} = 3,4 Hz; 2H; CH₂ éthyle); 7,98 (dd; ³J_{H-F6} = 10,8 Hz et ⁵J_{H-F8} = 2,0 Hz ; 1H; H-5); 8,62 (s; 1H; H-2); 14,63 (s; 1 H; COOH).
**RMN ¹³C** (CDCl₃) en ppm : 16,51 (d; ⁵J_{C-F8} ~ 5,4 Hz; CH₃ éthyle); 28,26 (s; 2 CH₂-S thiomorpholino); 53,52 (pseudo t; J ~ 3,8 Hz; CH₂-N thiomorpholino); 54,74 (d; ⁴J_{C-F8} = 16,8 Hz; CH2 éthyle); 108,36 (s; C-3); 108,68 (d; ²J_{C-F6} = 22,9 Hz; CH-5); 146,47 ( d; ¹J_{C-F8} = 247,6 Hz; C-8); 150,21 (s; CH-2); 155,48 (d; ¹J_{C-F6} = 244,4 Hz; C-6); 166,71 (s; COOH); 176,45 (s; CO).
**IR** (ATR) en cm⁻¹ : 3046 (valence C-H aromatiques et aliphatiques), 3100 à 2700 et 2700 à 2400 (valence acide O-H lié type dimère et valence C-H aromatiques et aliphatiques), 1710 (valence C=O acide type dimère), 1619 (valence C=O cétone conjuguée et valence asymétrique ion carboxylate OC-O), 1466 (valence C=C aromatiques), 1289 (valence C-F aromatique), 957 (H-O déformation hors du plan); 804 (valence C-H aromatique isolé).

### Acide 8-chloro-1-cyclopropyl-6-fluoro-1,4-dihydro-7-(4-méthyl-1-pipérazinyl)-4-oxoquinoline -3-carboxylique ; RN = [99696-26-1]

**pF :** 279°C
**RMN ¹H** (4% NaOD/D₂O) en ppm : 0,65 à 0,90 (m; 2H; cyclopropyle), 1,0 à 1,3 (m; 2H; cyclopropyle), 2,28 (s; 3H; CH₃ pipérazino); 2,4 à 2,7 (m; 4H; 2 CH₂ pipérazino); 3,1 à 3,4 (m; 4H; 2 CH₂ pipérazino); 4,1 (m; 1H; CH cyclopropyle), 7,69 (d; ³J_{H-F6} = 12,6 Hz; 1 H; H-5); 8,57 (s; 1H; H-2).
**RMN ¹³C** (4% NaOD/D₂O) en ppm : 13,24 (s; 2 CH₂ cyclopropyle), 42,73 (s; CH cyclopropyle), 47,61 (s; CH₃ pipérazino); 52,82 (s; 2 CH₂ pipérazino) ; 57,17 (s; 2 CH₂ pipérazino); 113,60 (d; ²J_{C-F6} = 23,7 Hz; CH-5); 119,00 (s; C-3); 121,22 (d; ⁴J_{C-F6} = 5,4 Hz; C-8a); 127,35 (d; ³J_{C-F6} = 6,9 Hz; C-4a); 139,85 (s; C-8); 144,98 (d; ²J_{C-F6} = 14,5 Hz; C-7); 154,14 (s; CH-2); 157,58 (d; ¹J_{C-F6} = 248,2 Hz; C-6); 173,88 (s; COOH); 177,53 (s; CO).
**IR** (ATR) en cm⁻¹ : 3600 à 3100 (valence O-H lié type dimère), 2700 à 2300 (valence O-H lié type dimère et valence C-H aromatiques et aliphatiques) 1716 (valence C=O acide type dimère), 1613 (valence C=O cétone conjuguée et valence asymétrique ion carboxylate O-C-O), 1458 - 1448 (valence C=C aromatiques), 1280 (valence C-F aromatique), 950 (H-O déformation hors du plan), 806 (valence C-H aromatique isolé).

### Acide 8-chloro-1-cyclopropyl-6-fluoro-1,4-dihydro-7-(4-éthyl-1-pipérazinyl)-4-oxo-quinoline-3-carboxylique ; RN = [116020-28-1].

**pF** : 200 °C
**RMN ¹H** (CDCl₃) en ppm : 0,9 à 1,05 (m; 2H; cyclopropyle); 1,16 (t; ³J = 7 Hz; 3H; CH₃ éthyle); 1,2 à 1,4 (pseudo q; 2H; cyclopropyle); 2,54 (q; ³J = 7 Hz; 2H; CH₂ éthyle); 2,55 à 2,75 (m; 4H; 2 CH₂ pipérazino); 3,35 à 3,55 (m; 4H; 2 CH₂ pipérazino); 4,36 (m; 1H; CH cyclopropyle); 7,98 (d; ³J_{H-F6} = 12,0 Hz; 1 H; H-5); 8,89 (s; 1 H; H-2); 13,5 (s large ; 1 H ; COOH).
**RMN ¹³C** (CDCl₃) en ppm : 11,44 (s; 2 CH₂ cyclopropyle); 11,97 (s; CH₃ éthyle); 41,32 (s; CH cyclopropyle); 51,25 (d; ⁴J_{C-F6} = 4,6 Hz; 2 CH₂ pipérazino); 52,59 (s; CH₂ éthyle) ; 53,26 (s; 2 CH₂ pipérazino); 108,60 (s; C-3); 111,66 (d; ²J_{C-F6} = 23,7 Hz; CH-5); 119,39 (d; ⁴J_{C-F6} = 5,5 Hz; C-8a); 123,33 (d; ³J_{C-F6} = 8,4 Hz; C-4a); 138,12 (s; C-8); 144,68 (d; ²J_{C-F6} = 13,8 Hz; C-7); 151,95 (s; CH-2); 156,27 (d; ¹J_{C-F6} = 252,0 Hz; C-6); 166,14 (s; COOH); 176,81 (d; ⁴J_{C-F6} = 3,0 Hz; CO).
**IR** (ATR) en cm⁻¹ : 3088 (valence C-H aromatiques et aliphatiques), 3000 à 2770 (valence O-H acide type dimère et valence C-H aromatiques et aliphatiques), 1723 (valence C=O acide type dimère), 1614 - 1601 (valence C=O cétone conjuguée et valence asymétrique ion carboxylate O-C-O), 1429 (valence C=C aromatiques); 1257 (valence C-F aromatique), 806 (valence C-H aromatique isolé).

## Revendications

1. Procédé de préparation d'un composé de formule (I) : 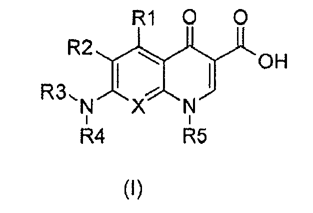 ou d'un hydrate ou d'un sel d'addition acide ou basique de celui-ci,
dans laquelle :
R₁ représente un groupement choisi parmi Hydrogène, Alkyle, ou NRR' ;
R₂ représente un atome de fluor ;
R₃, R₄ identiques ou différents
- représentent indépendamment un groupement Hydrogène, Alkyle, Cycloalkyle, Hydroxyle, Aralkyle, lesdits groupes Alkyle, Cycloalkyle, Aralkyle, pouvant être éventuellement substitués par un ou plusieurs groupes hydroxyles, ou NRR' ;
ou
- forment ensemble avec l'atome d'azote auquel ils sont attachés un groupement hétérocyclyle éventuellement substitué par un ou plusieurs groupes choisis parmi Alkyle, Hydroxyle, Alkoxy, -C(=O)Alkyle, NRR', =NOR, Aralkyle, Aryle, Hétéroaryle,
lesdits groupes Alkyle, Aryle et Hétéroaryles pouvant être éventuellement substitués par un ou plusieurs groupes choisis parmi Alkyle, Halogène, Perfluoroalkyle, Alkoxy, NRR';
R₅ représente un groupement choisi parmi Hydrogène, Alkyle, Cycloalkyle, Aryle, NR(CHO) ou NRR', lesdits groupes Alkyles et Aryles pouvant être éventuellement substitués par un ou plusieurs groupes choisis parmi Halogène ou Hydroxyle ;
X représente un groupement CR₈ ou un atome d'azote ;
R₈ représente un groupement choisi parmi Hydrogène, Halogène, Alkyle, ou Alkoxy, ou forme avec R₅ un groupement Hétérocyclyle, éventuellement substitué par un ou plusieurs groupes Alkyle ;
R, R' identiques ou différents représentent indépendamment un groupe Hydrogène ou Alkyle ;
ledit procédé comprenant :
i) la réaction d'un composé de formule (II) 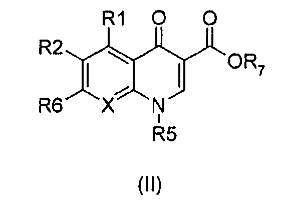 dans laquelle
R₁, R₂, R₅ et X sont tels que définis ci-dessus,
R₆ représente un atome d'Halogène ;
R₇ représente un groupement Alkyle ;
avec une amine de formule R₃R₄NH dans l'eau, en présence d'une base minérale ; et éventuellement
ii) la récupération du composé de formule (I) obtenu.

2. Procédé selon la revendication 1, dans lequel l'amine R₃R₄NH représente 1 à 5 équivalents en moles par rapport au composé de formule (II).

3. Procédé selon la revendication 1, dans lequel la base minérale est un carbonate ou hydrogénocarbonate de métal alcalin.

4. Procédé selon la revendication 3, dans laquelle la base minérale est choisie parmi K₂CO₃, KHCO₃, Na₂CO₃, ou NaHCO₃.

5. Procédé selon l'une quelconque des revendications 1 à 4, dans lequel R₆ représente un atome de fluor ou de chlore.

6. Procédé selon l'une quelconque des revendications 1 à 5, dans lequel R₃ et R₄ forment ensemble un groupe hétérocyclyle.

7. Procédé selon la revendication 6, dans lequel R₃ et R₄ forment un groupe pipérazinyle.

8. Procédé selon l'une quelconque des revendications 1 à 7, dans lequel le composé de formule (I) est la loméfloxacine.

9. Procédé selon l'une quelconque des revendications 1 à 8, dans lequel le composé de formule (I) obtenu est mis à réagir avec une solution d'acide chlorhydrique.

## Claims

1. Process for the preparation of a compound of the formula (I): 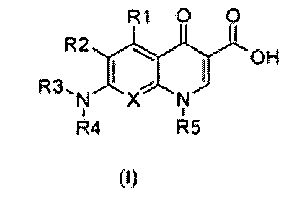 or of a hydrate or of an acidic or basic addition salt thereof, in which
R₁ represents a grouping chosen from hydrogen, alkyl or NRR';
R₂ represents a fluorine atom;
R₃, R₄ are identical or different and
- independently represent a hydrogen, alkyl, cycloalkyl, hydroxyl or aralkyl grouping, it being possible for the said alkyl, cycloalkyl and aralkyl groups to be optionally substituted by one or more hydroxyl or NRR' groups;
or
- together with the nitrogen atom to which they are bonded, form a heterocyclyl grouping optionally substituted by one or more groups chosen from alkyl, hydroxyl, alkoxy, -C(=O)alkyl, NRR', =NOR, aralkyl, aryl or heteroaryl,
it being possible for the said alkyl, aryl and heteroaryl groups to be optionally substituted by one or more groups chosen from alkyl, halogen, perfluoroalkyl, alkoxy or NRR';
R₅ represents a grouping chosen from hydrogen, alkyl, cycloalkyl, aryl, NR(CHO) or NRR', it being possible for the said alkyl and aryl groups to be optionally substituted by one or more groups chosen from halogen or hydroxyl;
X represents a CR₈ grouping or a nitrogen atom;
R₈ represents a grouping chosen from hydrogen, halogen, alkyl or alkoxy, or with R₅ forms a heterocyclyl grouping optionally substituted by one or more alkyl groups;
R, R' are identical or different and independently represent a hydrogen or alkyl group;
the said process comprising:
i) the reaction of a compound of the formula (II) 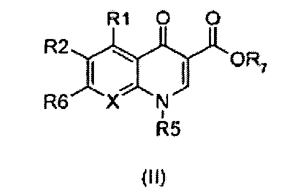 in which
R₁, R₂, R₅ and X are as defined above,
R₆ represents a halogen atom;
R₇ represents an alkyl grouping;
with an amine of the formula R₃R₄NH in water in the presence of a mineral base; and optionally
ii) isolation of the compound of the formula (I) obtained.

2. Process according to claim 1, in which the amine R₃R₄NH is present in an amount of 1 to 5 molar equivalents with respect to the compound of the formula (II).

3. Process according to claim 1, in which the mineral base is an alkali metal carbonate or bicarbonate.

4. Process according to claim 3, in which the mineral base is chosen from K₂CO₃, KHCO₃, Na₂CO₃ or NaHCO₃.

5. Process according to any one of claims 1 to 4, in which R₆ represents a fluorine or chlorine atom.

6. Process according to any one of claims 1 to 5, in which R₃ and R₄ together form a heterocyclyl group.

7. Process according to claim 6, in which R₃ and R₄ form a piperazinyl group.

8. Process according to any one of claims 1 to 7, in which the compound of the formula (I) is lomefloxacin.

9. Process according to any one of claims 1 to 8, in which the compound of the formula (I) obtained is reacted with a solution of hydrochloric acid.

## Patentansprüche

1. Verfahren zur Herstellung einer Verbindung der Formel (I) : 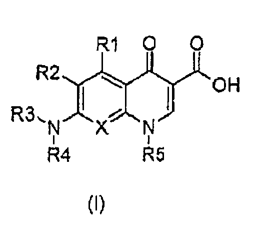 oder eines Hydrats oder eines Säureadditionssalzes oder Basensalzes derselben,
worin:
R₁ eine Gruppierung, ausgewählt aus Wasserstoff, Alkyl und NRR', darstellt;
R₂ ein Fluoratom darstellt;
R₃, R₄, die gleich oder verschieden sind,
- unabhängig Wasserstoff, eine Alkyl-, Cycloalkyl-, Hydroxyl-, Aralkylgruppierung darstellen, wobei die Alkyl-, Cycloalkyl-, Aralkylgruppen gegebenenfalls mit einer oder mehreren Hydroxyl- oder NRR'-Gruppen substituiert sein können;
oder
- zusammen mit dem Stickstoffatom, an das sie gebunden sind, eine Heterocyclylgruppierung bilden, die gegebenenfalls mit einer oder mehreren Gruppen, ausgewählt aus Alkyl, Hydroxyl, Alkoxy, -C(=O)Alkyl, NRR', =NOR, Aralkyl, Aryl, Heteroaryl, substituiert sind, wobei die Alkyl-, Aryl- und Heteroarylgruppen gegebenenfalls mit einer oder mehreren Gruppen, ausgewählt aus Alkyl, Halogen, Perfluoralkyl, Alkoxy, NRR', substituiert sein können;
R₅ eine Gruppierung, ausgewählt aus Wasserstoff, Alkyl, Cycloalkyl, Aryl, NR(CHO) und NRR', darstellt, wobei die Alkyl- und Arylgruppen gegebenenfalls mit einer oder mehreren Gruppen, ausgewählt aus Halogen und Hydroxyl, substituiert sein können;
X eine CR₈-Gruppierung oder ein Stickstoffatom darstellt;
R₈ eine Gruppe, ausgewählt aus Wasserstoff, Halogen, Alkyl und Alkoxy, darstellt oder mit R₅ eine Heterocyclylgruppierung, die gegebenenfalls mit einer oder mehreren Alkylgruppen substituiert ist, bildet;
R, R', die gleich oder verschieden sind, unabhängig Wasserstoff oder eine Alkylgruppe darstellen;
wobei das Verfahren umfasst:
i) Reaktion einer Verbindung der Formel (II) 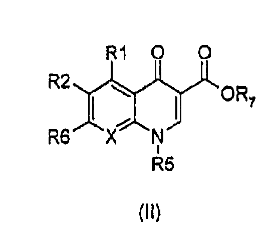 in der
R₁, R₂, R₅ und X wie vorstehend definiert sind,
R₆ ein Halogenatom darstellt;
R₇ eine Alkylgruppe darstellt;
mit einem Amin der Formel R₃R₄NH in Wasser in Gegenwart einer mineralischen Base und gegebenenfalls
ii) Isolierung der erhaltenen Verbindung der Formel (I).

2. Verfahren gemäß Anspruch 1, in dem das Amin R₃R₄NH 1 bis 5 Moläquivalente bezogen auf Verbindung der Formel (II) darstellt.

3. Verfahren gemäß Anspruch 1, in dem die mineralische Base ein Alkalimetallcarbonat oder -hydrogencarbonat ist.

4. Verfahren gemäß Anspruch 3, in dem die mineralische Base aus K₂CO₃, KHCO₃, Na₂CO₃ und NaHCO₃ ausgewählt wird.

5. Verfahren gemäß einem der Ansprüche 1 bis 4, in dem R₆ ein Fluor- oder Chloratom darstellt.

6. Verfahren gemäß einem der Ansprüche 1 bis 5, in dem R₃ und R₄ zusammen eine Heterocyclylgruppe bilden.

7. Verfahren gemäß Anspruch 6, in dem R₃ und R₄ eine Piperazinylgruppe bilden.

8. Verfahren gemäß einem der Ansprüche 1 bis 7, in dem die Verbindung der Formel (I) Lomefloxacin ist.

9. Verfahren gemäß einem der Ansprüche 1 bis 8, in dem die Verbindung der Formel (I), die erhalten wird, mit einer Salzsäurelösung reagieren gelassen wird.
